# EUROPÄISCHE PATENTSCHRIFT

(11) **EP 1 517 892 B1**
(45) Veröffentlichungstag und Bekanntmachung des Hinweises auf die Patenterteilung: **25.08.2010**
(21) Anmeldenummer: 03760591.2
(22) Anmeldetag: 04.06.2003
(51) Int. Cl.: C07D 205/08, A61K 31/397, A61P 3/06

(54) **RINGSUBSTITUIERTE DIPHENYLAZETIDINONE, VERFAHREN ZU DEREN HERSTELLUNG, DIESE VERBINDUNGEN ENTHALTENDE ARZNEIMITTEL UND DEREN VERWENDUNG**
RING-SUBSTITUTED DIPHENYL AZETIDINONES, METHOD FOR THE PRODUCTION THEREOF, MEDICAMENTS CONTAINING SAID COMPOUNDS, AND USE THEREOF
DIPHENYLAZETIDINONES A NOYAU SUBSTITUE, PROCEDES POUR LES PRODUIRE, MEDICAMENTS CONTENANT CES COMPOSES ET LEUR UTILISATION

(30) Priorität: 19.06.2002 DE 10227506
(43) Veröffentlichungstag der Anmeldung: 30.03.2005
(73) Patentinhaber: Sanofi-Aventis Deutschland GmbH, 65929 Frankfurt am Main (DE)
(72) Erfinder: JAEHNE, Gerhard, 65929 Frankfurt (DE); FRICK, Wendelin, 65510 Hünstetten-Beuerbach (DE); FLOHR, Stefanie, CH-4054 Basel (CH); LINDENSCHMIDT, Andreas, 65812 Bad Soden (DE); GLOMBIK, Heiner, 65719 Hofheim (DE); KRAMER, Werner, 55130 Mainz-Laubenheim (DE); HEUER, Hubert, 55270 Schwabenheim (DE); SCHAEFER, Hans-Ludwig, 65239 Hochheim (DE)
(86) Internationale Anmeldenummer: PCT/EP2003/005815
(87) Internationale Veröffentlichungsnummer: WO 2004/000804

(56) Entgegenhaltungen:
- WO-A-02/18432
- WO-A-96/19450
- WO-A-97/16455

## Beschreibung

Die Erfindung betrifft ringsubstituierte Diphenylazetidinone, deren physiologisch verträgliche Salze sowie physiologisch funktionelle Derivate.

Es sind bereits Diphenylazetidinone (wie z.B. Ezetimibe) sowie deren Verwendung zur Behandlung von Hyperlipidämie sowie Arteriosklerose und Hypercholesterinämie beschrieben worden [vgl. Drugs of the Future 2000, 25(7):679-685) und US 5,756,470].

Der Erfindung lag die Aufgabe zugrunde, weitere Verbindungen zur Verfügung zu stellen, die eine therapeutisch verwertbare hypolipidämische Wirkung entfalten. Insbesondere bestand die Aufgabe darin, neue Verbindungen zu finden, die gegenüber den im Stand der Technik beschriebenen Verbindungen, sehr gering resorbierbar sind. Unter sehr gering resorbierbar wird eine intestinale Resorption kleiner 10%, bevorzugt kleiner oder gleich 5% verstanden.

Die neuen Verbindungen sollen insbesonders eine geringere Resorption als Ezetimibe auf weisen.

Bei geringerer Resorption zeigen pharmazeutische Wirkstoffe in der Regel deutlich weniger Nebenwirkungen.

Die Erfindung betrifft daher Verbindungen der Formel I, 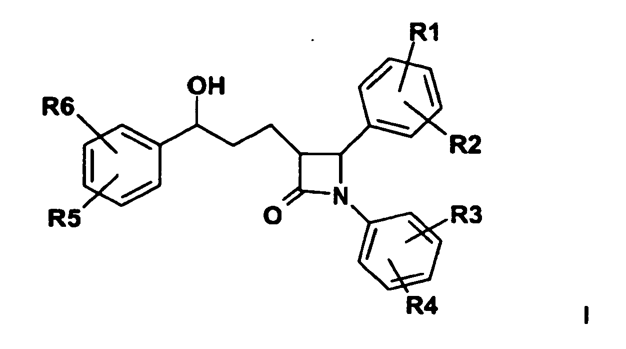 worin bedeuten
- R2, R4, R5, R6: unabhängig voneinander H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O- (C₁-C₆)-Alkyl, wobei in den Alkylresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂, S-(C₁-C₆)- Alkyl, S-(CH₂)ₙ-Phenyl, SO-(C₁-C₆)-Alkyl, SO-(CH₂)ₙ-Phenyl, SO₂- (C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O- (CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁- C₆)-Alkyl)₂, SO₂-CH₃, COOH, COO-(C₁-C₆)-Alkyl, CONH₂;
- R1, R3: unabhängig voneinander -(CH₂)₀₋₁-NH-(C=O)₀₋₁-(C₀-C₂₅)-Alkylen- (C=O)₀₋₁-N(R13)₀₋₁-(LAG) oder -(CH₂)₀₋₁-(C=O)₀₋₁-NH-(C₀-C₂₅)- Alkylen-(C=O)₀₋₁-N(R13)₀₋₁-(LAG) hat; worin ein oder mehrere C- Atome des Alkylenrests, durch bis zu dreifach mit R7 substituierte Aryl- oder Heteroarylreste oder durch bis zu dreifach mit R7substituierte (C₃-C₁₀)-Cycloalkyl- oder Heterocycloalkylreste ersetzt sind und worin ein oder mehrere C-Atome des Alkylenrests durch O-Atome ersetzt sein können; H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂- C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂ , S-(C₁-C₆)- Alkyl, S-(CH₂)ₙ-Phenyl, SO-(C₁-C₆)-Alkyl, SO-(CH₂)ₙ-Phenyl, SO₂- (C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O- (CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁- C₆)-Alkyl)₂, SO₂-CH₃, COOH, COO-(C₁-C₆)-Alkyl, CONH₂;
- R7: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)- Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ- Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)- Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)- Alkyl, CONH₂;
- LAG: Monozuckerest, ein acyclischer, mono-, bi- oder tricyclischer Trialkylammoniumalkyl-Rest, eine Sulfonsäure oder eine Carbonsäure,
- R13: H oder CH₃;
und wobei immer mindestens einer der Reste R1 oder R3 die Bedeutung -(CH₂)₀₋₁-NH-(C=O)₀₋₁-(C₀-C₂₅)-Alkylen-(C=O)₀₋₁-N(R13)₀₋₁-(LAG) oder -(CH₂)₀₋₁-(C=O) ₀₋₁-NH-(C₀-C₂₅)-Alkylen-(C=O)₀₋₁-N(R13)₀₋₁-(LAG) hat und ein oder mehrere C-Atome des Alkylenrests durch bis zu dreifach substituierte Aryl- oder Heteroarylreste oder durch bis zu dreifach substituierte (C₃-C₁₀)-Cycloalkyl- oder Heterocycloalkylreste ersetzt sind und zusätzlich durch -S(O)ₙ-, mit n = 0 - 2, -O-, -(C=O)-, -N(CH₃)-, -N(Phenyl)- oder -NH- ersetzt sein können,
besitzen muß;
sowie deren physiologisch verträglichen Salze.

Unter einem Arylrest wird ein Phenyl-, Naphthyl-, Biphenyl-, Tetrahydronaphthyl-, alpha- oder beta-Tetralon-, Indanyl- oder Indan-1-on-ylrest verstanden.

Unter einem Heteroarylrest wird ein Pyridyl-, Pyrimidinyl-, Pyrazinyl-, Pyridazinyl-, Pyridazin-3-on-yl-, Pyrrolyl-, Imidazolyl-, Pyrazolyl-, 1,2,4-Triazolyl-, Indolyl-, Benzimidazolyl-, Thienyl-, Furyl-, Thiazolyl-, Oxazolyl-, Isoxazolyl- oder Isothiazolylrest verstanden.

Unter Heterocycloalkylresten werden (C₃-C₁₀)-Cycloalkylreste verstanden, bei denen wenigstens ein und bis zu drei Kohlenstoffatome unabhängig voneinander durch NR8, O oder S(O)ₙ , wobei n = 0 - 2 ist, ersetzt ist. Beispiele sind der Azetidinyl-, Pyrrolidinyl-, Piperidinyl-, Morpholinyl-, 1,4-Dioxanyl-, Tetrahydrofuryl-, Piperazinyl- oder Thiepanylrest.

Unter einem acyclischen Trialkylammonium-Rest wird folgende Gruppe verstanden 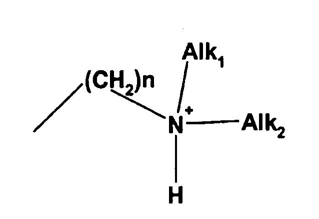 worin n = 0 bis 10 sein kann und Alk₁ und Alk₂ unabhängig voneinander jeweils einen geraden oder verzweigten Alkylrest mit 1 bis 20 Kohlenstoffatomen bedeutet.

Unter einem acyclischen Trialkylammoniumalkyl-Rest wird folgende Gruppe verstanden 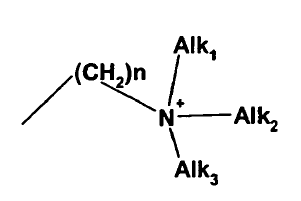 worin n = 0 bis 10 sein kann und Alk₁, Alk₂, Alk₃ unabhängig voneinander jeweils einen geraden oder verzweigten Alkylrest mit 1 bis 20 Kohlenstoffatomen bedeutet.

Unter einem mono- oder bi- oder tricyclischen Trialkylammonium-Rest werden z.B. Reste wie 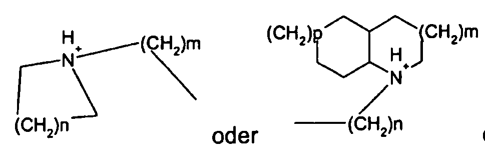 oder 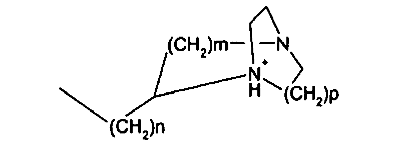 verstanden, wobei n, m und p unabhängig voneinander 0 - 10 sein kann und eine oder mehrere CH₂-Gruppen unabhängig voneinander durch O, S(O)ₙ, wobei n = 0 - 2 sein kann, NH, N-(C₁-C₁₀)-Alkyl, N-Phenyl oder N-CH₂-Phenyl ersetzt sein können.

Unter einem mono- oder bicyclischen Trialkylammoniumalkyl-Rest werden z.B. Reste wie 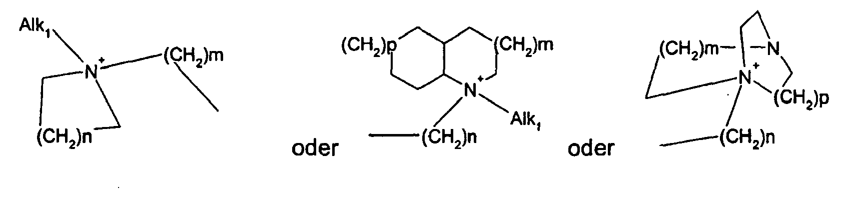 oder 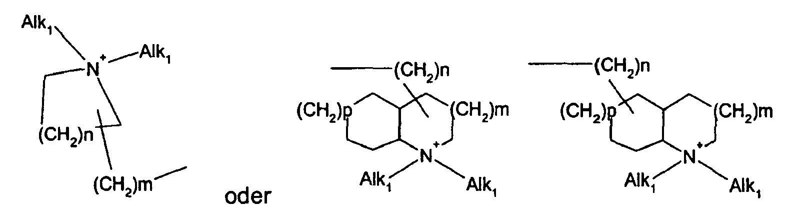 oder 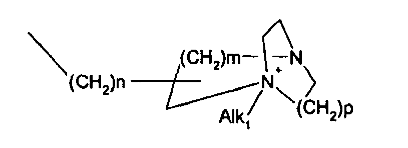 verstanden, wobei n, m und p unabhängig voneinander 0 -10 sein kann und eine oder mehrere CH₂-Gruppen unabhängig voneinander durch O, S(O)ₙ, wobei n = 0 - 2 sein kann, NH, N-(C₁-C₁₀)-Alkyl, N-Phenyl oder N-CH₂-Phenyl ersetzt sein können und Alk₁einen geraden oder verzweigten Alkylrest mit 1 bis 20 Kohlenstoffatomen bedeutet.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung sind Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Verbindungen der Formel I und deren pharmazeutisch verträgliche Salze und physiologisch funktionelle Derivate stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen.

Die Verbindung(en) der Formel (I) können auch in Kombination mit weiteren Wirkstoffen verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im bereich von 0.1 mg bis 100 mg (typischerweise von 0,1 mg und 50 mg) pro Tag pro Kilogramm Körpergewicht, z.B: 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Diphenylazetidinon-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylzäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Als weitere Wirkstoffe für die Kombinationspräparate sind geeignet: Alle Antidiabetika, die in der Roten Liste 2001, Kapitel 12 genannt sind. Sie können mit den erfindungsgemäßen Verbindungen der Formel I insbesonders zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Antidiabetika umfassen Insulin und Insulinderivate, wie z.B. Lantus^{®} oder HMR 1964, GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguadine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlididämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Bay 13-9952, BMS-201038, R-103757, verabreicht.

Bei einer Ausführungsform der Erfindung werden die Verbindungen der Formel I in Kombination mit Gallensäureresorptionsinhibitor , wie z.B. HMR 1453, verabreicht.

Bei einer Ausführungsform der Erfindung werden die Verbindungen der Formel I in Kombination mit einem CETP-Inhibitor, wie z.B. Bay 194789, verabreicht.

Bei einer Ausführungsform der Erfindung werden die Verbindungen der Formel I in Kombination mit einem polymeren Gallensäureadsorber, wie z.B. Cholestyramin, Colesolvam, verabreicht.

Bei einer Ausführungsform der Erfindung werden die Verbindungen der Formel I in Kombination mit einem LDL-Rezeptorinducer, wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen synthetase inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Gliclazid, verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Gliazid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Agonisten, NPY-Agonisten, MC-3- und MC-4-Agonisten, Orexin-Agonisten, H3-Agonisten, TNF-Agonisten, CRF-Agonisten, CRF BP-Antagonisten, Urocortin-Agonisten, β3-Agonisten, MCH (Melaninkonzentrierendes Hormon) Antagonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormon, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen, wie z.B. Caromax^{®} verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden. Die Kombination von Verbindungen der Formel I mit Caromax^{®} zeichnet sich neben einer Wirkverbesserung, insbesonders in der LDL-Cholesterinsenkung, gegenüber den Einzelwirkstoffen, auch durch Ihre verbesserte Verträglichkeit aus.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Bevorzugt sind racemische als auch enantiomerenreine Verbindungen der Formel I mit folgender Struktur:

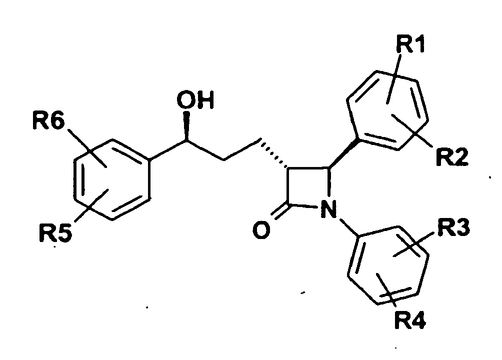

Unter Zuckerresten werden Verbindungen verstanden, die sich von Aldosen und Ketosen mit 3 bis 7 Kohlenstoffatomen ableiten, die der D- oder L-Reihe angehören können; dazu gehören auch Aminozucker, Zuckeralkohole oder Zuckersäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure, Mannonsäure, Glucamin, 3-Amino-1,2-propandiol, Glucarsäure und Galaktarsäure.

Mit Dizucker sind Saccharide gemeint, die aus zwei Zuckereinheiten bestehen. Di-, Tri-, oder Tetrasaccharide entstehen durch acetalartige Bindung von 2 oder mehreren Zuckern. Die Bindungen können dabei in der α- oder β-Form auftreten. Beispielhaft seien genannt Laktose, Maltose und Cellobiose.

Wenn der Zucker substituiert ist, so erfolgt die Substitution bevorzugt am Wasserstoffatom einer OH-Gruppe des Zuckers.

Für die Hydroxygruppen der Zucker kommen im wesentlichen folgende Schutzgruppen in Frage: Benzyl-, Acetyl-, Benzoyl-, Pivaloyl-, Trityl-, tert.-Butyldimethylsilyl-, Benzyliden-, Cyclohexyliden- oder Isopropylidenschutzgruppen.

Mit dem Begriff Aminosäuren bzw. Aminosäurereste sind z.B. die stereoisomeren Formen, d.h. D- oder L-Formen, folgender Verbindungen gemeint:

| | | |
|---|---|---|
| Alanin | Glycin | Prolin |
| Cystein | Histidin | Glutamin |
| Asparaginsäure | Isoleucin | Arginin |
| Glutaminsäure | Lysin | Serin |
| Phenylalanin | Leucin | Threonin |
| Tryptophan | Methionin | Valin |
| Tyrosin | Asparagin | |

| | |
|---|---|
| 2-Aminoadipinsäure | 2-Aminoisobuttersäure |
| 3-Aminoadipinsäure | 3-Aminoisobuttersäure |
| beta-Alanin | 2-Aminopimelinsäure |
| 2-Aminobuttersäure | 2,4-Diaminobuttersäure |
| 4-Aminobuttersäure | Desmosin |
| Piperidincarbonsäure | 2,2-Diaminopimelinsäure |
| 6-Aminocapronsäure | 2,3-Diaminopropionsäure |
| 2-Aminoheptansäure | N-Ethylglycin |
| 2-(2-Thienyl)-glycin | 3-(2-Thienyl)-alanin |
| Penicillamin | Sarkosin |
| N-Ethylasparagin | N-Methylisoleucin |
| Hydroxylysin | 6-N-Methyllysin |
| allo-Hydroxylysin | N-Methylvalin |
| 3-Hydroxyprolin | Norvalin |
| 4-Hydroxyprolin | Norleucin |
| Isodesmosin | Ornithin |
| allo-Isoleucin | |
| N-Methylglycin | |

Die Kurzschreibweise der Aminosäuren erfolgte nach der allgemein üblichen Schreibweise (vgl. Schröder, Lübke, The Peptides, Band I, New York 1965, Seiten XXII-XXIII; Houben-Weyl, Methoden der Organischen Chemie, Band XV/1 und 2, Stuttgart 1974). Die Aminosäure pGlu steht für Pyroglutamyl, Nal für 3-(2-Naphthyl)-alanin, Azagly-NH₂ für eine Verbindung der Formel NH₂-NH-CONH₂ und D-Asp für die D-Form von Asparaginsäure. Peptide sind ihrer chemischen Natur nach Säureamide und zerfallen bei der Hydrolyse in Aminosäuren.

Unter Oligopeptid versteht man Peptide, die aus 2 bis 9 der oben genannten Aminosäuren aufgebaut sind.

Geeignete Schutzgruppen (siehe z.B. T.W. Greene, "Protective Groups in Organic Synthesis") für Aminosäuren sind in erster Linie: Arg(Tos), Arg(Mts), Arg(Mtr), Arg(PMV), Asp(OBzl), Asp(OBut), Cys(4-MeBzl), Cys(Acm), Cys(SBut), Glu(Obzl), Glu(Obut), His(Tos), His(Fmoc), His(Dnp), His(Trt), Lys(Cl-Z), Lys(Boc), Met(O), Ser(Bzl), Ser(But), Thr(Bzl), Thr(But), Trp(Mts), Trp(CHO), Tyr(Br-Z), Tyr(Bzl) oder Tyr(But) eingesetzt werden.

Als Aminoschutzgruppen werden bevorzugt der durch katalytische Hydrierung abspaltbare Benzyloxycarbonyl-(Z-)Rest, der durch schwache Säuren abspaltbare 2-(3,5-Dimethyloxyphenyl)propyl(2)oxycarbonyl (Ddz-) oder Trityl-(Trt)-Rest, der durch Säuren wie 3M Salzsäure abspaltbare t-Butylcarbamat (BOC-)-Rest und der durch sekundäre Amine abspaltbare 9-Fluorenylmethyloxycarbonyl- (Fmoc)-Rest herangezogen.

Die Erfindung betrifft weiterhin ein Verfahren zur Herstellung von Diphenylazetidinonderivaten der Formel I. 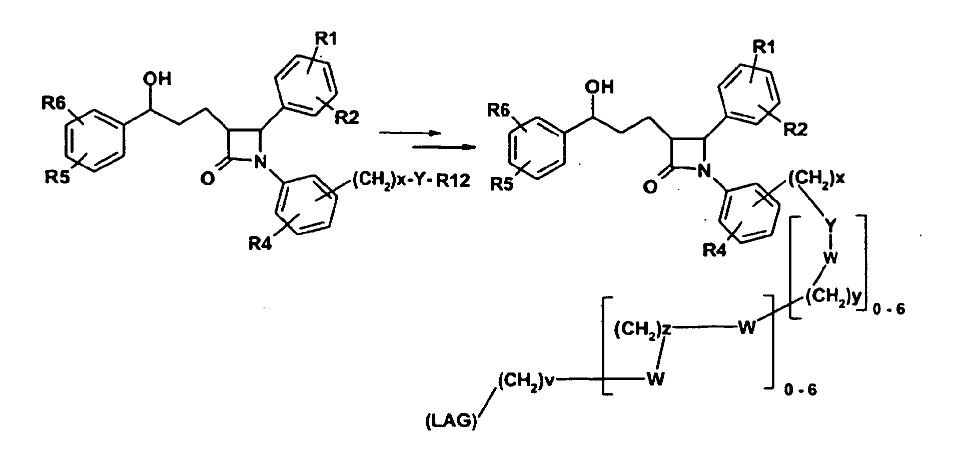 Y kann S, O, (C=O), (C=S), CH=CH, C≡C, N((C₁-C₆)-Alkyl), N(Phenyl), N((C₁-C₆)-Alkyl-Phenyl), N(CO-(CH₂)₁₋₁₀-COOH) oder NH bedeuten;
R12 kann H oder im Falle, dass Y = (C=O) oder (C=S) ist, OH bedeuten;
W kann, unabhängig von Y, bis zu dreifach substituierter Aryl- oder Heteroarylrest oder bis zu vierfach substituierter (C₃-C₁₀)-Cycloalkyl- oder Heterocycloalkylrest oder -S(O)ₙ-, mit n = 0 - 2, -O-, -(C=O)-, -(C=S)-, -CH=CH-, -C≡C-, -N((C₁-C₆)-Alkyl)-, -N(Phenyl), -N((C₁-C₆)-Alkyl-Phenyl)-, -N(CO-(CH₂)₁₋₁₀-COOH)- oder -NH- oder eine Bindung bedeuten;
v, x, y und z können unabhängig voneinander 0 bis 10 bedeuten.

Die Verknüpfung von -(CH₂)x-Y-R12 in Verbindung II kann alternativ auch an einem der anderen beiden Phenylringen sein.

Das Verfahren zur Herstellung der Verbindungen der Formel I ist z.B. **dadurch gekennzeichnet, daß** man ein Amin der Formel II mit einem Alkylierungs- oder einem Acylierungsreagenz umsetzt, das bevorzugt in omega-Position eine weitere Funktionalität - evtl. in geschützter Form - trägt. Diese wird (nach Entschützung) zur Anknüpfung der (LAG) verwendet, beispielsweise unter Ausbildung von Ether-, Amin oder Amidbindunger.

Die nachfolgenden Beispiele dienen zur näheren Erläuterung der Erfindung, ohne dieselbe auf in den Beispielen beschriebene Produkte und Ausführungsformen einzuschränken.

### Beispiel I

### 2,3,4,5,6-Pentahydroxy-hexylamid der 4-(4-{4-[3-[3-(4-Fluor-phenyl)-3-hydroxypropyl]-2-(4-methoxy-phenyl)-4-oxo-azetidin-1-yl]-benzylcarbamoyl}-phenoxy)-benzoesäure (7):

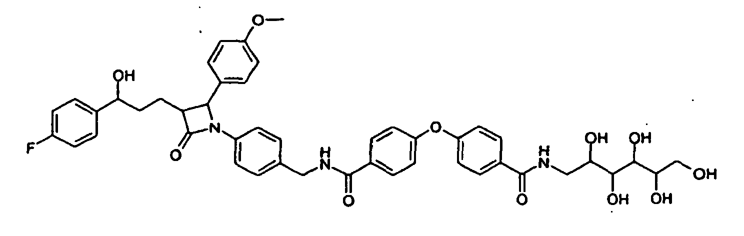

### a) 3-[5-(tert-Butyl-dimethyl-silanyloxy)-5-(4-fluor-phenyl)-pentanoyl]-4-phenyl-oxazolidin-2-on (1):

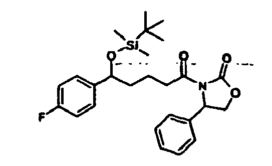

27 g 3-[5-(4-Fluor-phenyl)-5-hydroxy-pentanoyl]-4-phenyl-oxazolidin-2-on werden mit 13,6 g Tert.-Butyl-Dimethylsilylchlorid und 10,2 g Imidazol in 36 ml Dimethylformamid gelöst und 90 min. bei 60°C gerührt. Nach Beendigung der Reaktion wird das Gemisch in Essigsäureethylester gelöst und zweimal mit Wasser ausgeschüttelt. Die organische Phase wird über Magnesiumsulfat getrocknet, filtriert und im Vakuum eingeengt. Man erhält 3-[5-(tert-Butyldimethyl-silanyloxy)-5-(4-fluor-phenyl)-pentanoyl]-4-phenyl- oxazolidin-2-on (1) mit dem Molekulargewicht 471,65 (C₂₆H₃₄FNO₄Si); MS (ESI): 340.28 (MH⁺ - HOSi(CH₃)₂C(CH₃)₃).

### b) 4-[5-(tert-Butyl-dimethyl-silanyloxy)-5-(4-fluor-phenyl)-1-(4-methoxy-phenyl)-2-(2-oxo- 4-phenyl-oxazolidin-3-carbonyl)-pentylamino]-benzonitril (2):

16,2 g 3-[5-(tert-Butyl-dimethyl-silanyloxy)-5-(4-fluor-phenyl)-pentanoyl]-4-phenyl-oxazolidin-2-on (1) werden in 350 ml Dichlormethan gelöst. Die Lösung wird mit 19,8 ml Hünig Base und mit 10,14 g 4-[(4-Methoxy-phenylimino)-methyl]-benzonitril versetzt und auf -10°C gekühlt. Zur gekühlten Lösung fügt man 8,52 ml Trimethylsilyltriflat hinzu und rührt 30 min. bei -10°C. Die Lösung wird nun auf -30°C abgekühlt, und es werden 44 ml Titantetrachloridlösung zugegeben. Die Reaktionsmischung wird 2 h bei -30 bis-40°C gerührt. Danach lässt man die Lösung sich auf Raumtemperatur erwärmen, wäscht die Reaktionslösung nacheinander mit 200 ml 2N Schwefelsäure, 300 ml 20%iger Natriumhydrogensulfitllösung und ges. Kochsalzlösung. Die organische Phase wird über Magnesiumsulfat getrocknet, im Vakuum eingeengt und der Rückstand wird über Kieselgel mit n-Heptan/Essigsäureethylester 3/1 gereinigt. Man erhält 4-[5-(tert-Butyl-dimethyl-silanyloxy)-5-(4-fluor-phenyl)-1-(4-methoxy-phenyl)-2-(2-oxo- 4-phcnyl-oxazolidin-3-carbonyl)-pertylamino]-benzenitäl (2) mit dem Molekulargewicht 707,93 (C₄₁H₄₆FN₃O₅Si); MS (ESI): 590.51 (MH⁺ - C₇H₅N₂).

### c) 4-[3-[3-(tert-Butyl-dimethyl-silanyloxy)-3-(4-fluor-phenyl)-propyl]-2-(4-methoxy- phenyl)-4-oxo-azetidin-1-yl]-benzonitril (3):

13,2 g 4-[5-(tert-Butyl-dimethyl-silanyloxy)-5-(4-fluor-phenyl)-1-(4-methoxy-phenyl)-2-(2-oxo- 4-phenyl-oxazolidin-3-carbonyl)-pentylamino]-benzonitril (2) werden in 380 ml Methyl-tert.-Butylether gelöst, mit 18,6 ml N,O-Bis(trimethylsilyl)-acetamid und 1,86 ml einer 1 M Lösung von Tetrabutylammoniumfluorid in Tetrahydrofuran versetzt und 2 h bei Raumtemperatur gerührt. Nach Beendigung der Reaktion fügt man 10 ml Essigsäure zu, engt die Reaktionsmischung im Vakuum ein und reinigt den Rückstand über Kieselgel mit Toluol/Essigsäureethylester 50/1. Man erhält 4-[3-[3-(tert-Butyl-dimethyl-silanyloxy)-3-(4-fluor-phenyl)-propyl]-2-(4-methoxy-phenyl)-4-oxo-azetidin-1-yl]-benzonitril (3) mit dem Molekulargewicht 544,75 (C₃₂H₃₇FN₂O₃Si); MS (ESI): 545.56 (M+H⁺).

### d) 4-[3-[3-(4-Fluor-phenyl)-3-hydroxy-propyl]-2-(4-methoxy-phenyl)-4-oxo-azetidin-1-yl]- benzonitril (4):

3.5 g 4-[3-[3-(tert-Butyl-dimethyl-silanyloxy)-3-(4-fluor-phenyl)-propyl]-2-(4-methoxy- phenyl)-4-oxo-azetidin-1-yl]-benzonitril (3) werden in 65 ml Tetrahydrofuran gelöst, mit 0,74 ml Essigsäure und 8,03 ml einer 1 M Lösung von Tetrabutylammoniumfluorid in Tetrahydrofuran versetzt und 2 h bei Raumtemperatur gerührt. Danach werden 4,82 ml der Tetrabutylammoniumfluorid-Lösung nachgegeben und weitere 3 h bei Rückflusstemperatur gerührt. Die abgekühlte Reaktionsmischung wird im Vakuum eingeengt, und der Rückstand wird chromatographisch über Kieselgel mit n-Heptan/Essigsäureethylester 2/1 gereinigt. Man erhält 4-[3-[3-(4-Fluor-phenyl)-3-hydroxy-propyl]-2-(4-methoxy-phenyl)-4-cxo-azatdin-1-yl]- benzonitril (4) mit dem Molekulargewicht 430,48 (C₂₆H₂₃FN₂O₃); MS (ESI): 431.24 (M+H⁺).

### e) 1-(4-Aminomethyl-phenyl)-3-[3-(4-fluor-phenyl)-3-hydroxy-propyl]-4-(4-methoxy-phenyl)- azetidin-2-on (5):

1,22 g 4-[3-[3-(4-Fluor-phenyl)-3-hydroxy-propyl]-2-(4-methoxy-phenyl)-4-oxo-azetidin-1-yl]- benzonitril (4) werden in 90 ml Ethanol gelöst, mit 10 ml konz. Ammoniaklösung und einem Überschuß Raney-Nickel versetzt und 8 h bei 60°C und einem Druck von 10 bar Wasserstoff gerührt. Die Reaktionsmischung kühlt über Nacht auf Raumtemperatur ab; anderntags wird vom Katalysator abgetrennt, das Filtrat im Vakuum eingeengt und der Rückstand chromatographisch über Kieselgel mit Dichlormethan/Methanol/AmmoniakLösung 10/1/0.1 gereinigt. Man erhält 1-(4-Aminomethyl-phenyl)-3-[3-(4-fluor-phenyl)-3-hydroxy-propyl]-4-(4-methoxy-phenyl)- azetidin-2-on (5) mit dem Molekulargewicht 434,51 (C₂₆H₂₇FN₂O₃); MS (ESI): 418.2 (MH⁺ - NH₃).

### f) 2,3,4,5,6-Pentahydroxy-hexylamid der 4,4'-Oxybisbenzoesäure (6):

0,52 g 4,4'-Oxybisbenzoesäure und 0,36 g D-Glucamin werden bei Raumtemperatur in 10 ml trockenem Dimethylformamid suspendiert, mit 0.31 g HOBt und 0.39 g EDC versetzt und 12 h bei Raumtemperatur gerührt. Die Reaktionsmischung wird vollständig eingeengt und im Hochvakuum getrocknet. Der Rückstand wird gründlich mit Wasser verrührt, die enstandene Suspension wird filtriert, der Rückstand wird in Methanol verrührt und erneut filtriert. Das Filtrat wird am Rotationsverdampfer auf die Hälfte des Volumens konzentriet und die Lösung im Eisbad gekühlt. Der gebildete Niederschlag wird abgesaugt, mit wenig eiskaltem Methanol gewaschen und im Vakuum getrocknet. Man erhält das 2,3,4,5,6-Pentahydroxy-hexylamid der 4,4'-Oxybisbenzoesäure (6) mit dem Molekulargewicht 421,40 (C₂₀H₂₃NO₉); MS (ESI): 422,28 (MH⁺).

### g) 2,3,4,5,6-Pentahydroxy-hexylamid der 4-(4-{4-[3-[3-(4-Fluor-phenyl)-3-hydroxy-propyl]-2-(4-methoxy-phenyl)-4-oxo-azetidin-1-yl]-benzylcarbamoyl}-phenoxy)-benzoesäure (7):

87 mg der Verbindung wie unter e) hergestellt und 90 mg der Verbindung wie unter f) hergestellt werden bei Raumtemperatur in 3 ml trockenem Dimethylformamid gelöst, mit 31 mg HOBt und 39 mg EDC versetzt und 12 h bei Raumtemperatur gerührt. Die Reaktionsmischung wird im Hochvakuum vollständig eingeengt, und der Rückstand wird in Dichlormethan verrührt, abgesaugt, mit Dichlormethan gewaschen und im Vakuum getrocknet. Man erhält das 2,3,4,5,6-Pentahydroxy-hexylamid der 4-(4-{4-[3-[3-(4-Fluor-phenyl)-3-hydroxy-propyl]-2-(4-methoxy-phenyl)-4-oxo-azetidin-1-yl]-benzylcarbamoyl}-phenoxy)-benzoesäure (7) mit dem Molekulargewicht 837,90 (C₄₆H₄₈FN₃O₁₁); MS (ESI): 838,39 (MH⁺).

### Beispiel II

### 2,3,4,5,6-Pentahydroxy-hexylamid der 4-(4-{4-[3-(3-Hydroxy-3-phenyl-propyl)-2-oxo-4-phenyl-azetidin-1-yl]-benzylcarbamoyl}- phenoxy)-benzoesäure (9):

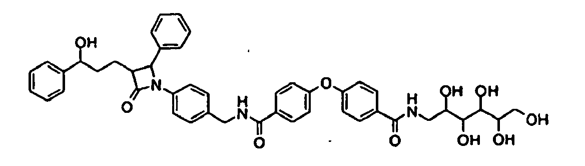

### a) 1-(4-Aminomethyl-phenyl)-3-(3-hydroxy-3-phenyl-propyl)-4-phenyl-azetidin-2-on (8):

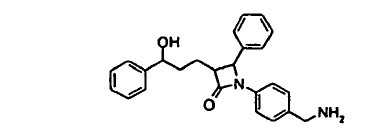

Diese Verbindung wird, wie vorn für 1-(4-Aminomethyl-phenyl)-3-[3-(4-fluor-phenyl)-3-hydroxy-propyl]-4-(4-methoxy-phenyl)- azetidin-2-on beschrieben, hergestellt, mit dem Unterschied, dass 3-[5-(tert-Butyl-dimethyl-silanyloxy)-5-phenyl-pentanoyl]-4-phenyl-oxazolidin-2-on und 4-(Benzyliden-amino)-benzonitril Einsatz finden. Man erhält 1-(4-Aminomethyl-phenyl)-3-(3-hydroxy-3-phenylpropyl)-4-phenyl-azetidin-2-on (8) mit dem Molekulargewicht 386,50 (C₂₅H₂₆N₂O₂): MS (ESI): 370,2 (MH⁺-NH₃).

### b) 2,3,4,5,6-Pentahydroxy-hexylamid der 4-(4-{4-[3-(3-Hydroxy-3-phenylpropyl)-2-oxo-4-phenyl-azetidin-1-yl]-benzylcarbamoyl}- phenoxy)-benzoesäure (9):

Das Benzylamin aus IIa wird mit dem 2,3,4,5,6-Pentahydroxy-hexylamid der 4,4'-Oxybisbenzoesäure aus If wie im Beispiel I beschrieben umgesetzt. Man erhält das 2,3,4,5,6-Pentahydroxy-hexylamid der 4-(4-{4-[3-(3-Hydroxy-3-phenylpropyl)-2-oxo-4-phenyl-azetidin-1-yl]-benzylcarbamoyl}- phenoxy)-benzoesäure (9) mit dem Molekulargewicht 789,89 (C₄₅H₄₇N₃O₁₀); MS (ESI): 790,26 (MH⁺).

### Beispiel III

### 2,3,4,5,6-Pentahydroxy-hexylamid der 4-(4-{4-[3-[3-(4-Fluor-phenyl)-3-hydroxypropyl]-2-(4-methoxy-phenyl)-4-oxo-azetidin-1-yl]-benzylcarbamoyl}-benzyl)-benzoesäure (10):

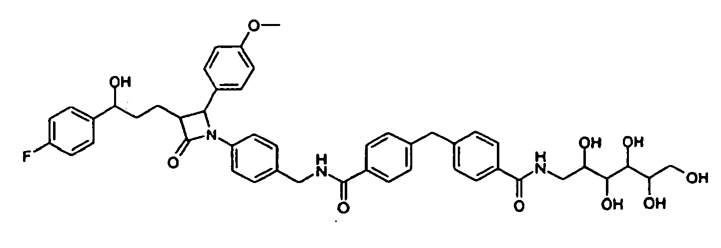

Die Verbindung des Beispieles III wird analog der Vorgehensweise des Beispiels I gewonnen, mit dem Unterschied dass das 2,3,4,5,6-Pentahydroxy-hexylamid der Diphenylmethan-4,4'-dicarbonsäure eingesetzt wird. Man erhält das 2,3,4,5,6-Pentahydroxy-hexylamid der 4-(4-{4-[3-[3-(4-Fluor-phenyl)-3-hydroxy-propyl]-2-(4-methoxy-phenyl)-4-oxo-azetidin-1-yl]-benzylcarbamoyl}-benzyl)-benzoesäure (10) mit dem Molekulargewicht 835,93 (C₄₇H₅₀FN₃O₁₀); MS (ESI): 836,18 (MH⁺).

### Beispiel IV

### 1-[4-(4-{1-(4-Fluor-phenyl)-3-[3-(4-fluor-phenyl)-3-hydroxy-propyl]-4-oxo-azetidin-2- yl}-phenoxymethyl)-benzyl]-4-aza-1-azonia-bicyclo[2.2.2]octan Bromid (13):

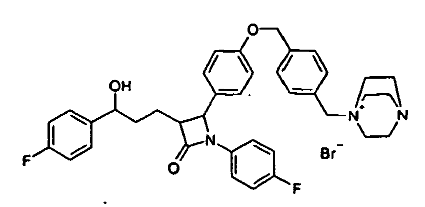

### a) 4-[4-(4-Brommethyl-benzyloxy)-phenyl]-1-(4-fluor-phenyl)-3-[3-(4-fluor-phenyl)-3- hydroxy-propyl]-azetidin-2-on (12):

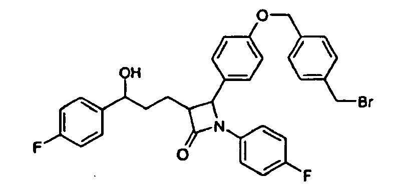

3,0 g 1-(4-Fluor-phenyl)-3-[3-(4-fluor-phenyl)-3-hydroxy-propyl]-4-(4-hydroxyphenyl)- azetidin-2-on (11) werden mit 7,0 g 1,4-Bis-brommethyl-benzol und 5,0 g Kaliumcarbonat in 100 ml Dimethylformamid gelöst und 90 min. bei Raumtemperatur gerührt. Nach Beendigung der Reaktion wird das Gemisch in Essigsäureethylester gelöst und zweimal mit Wasser ausgeschüttelt. Die organische Phase wird über Magnesiumsulfat getrocknet, filtriert und im Vakuum eingeengt. Der Rückstand wird mit Flashchromatographie (n-Heptan/Ethylacetat) gereinigt. Ausbeute 3,2 g farbose Kristalle (12) mit dem Molekulargewicht 592,49 (C₃₂H₂₈BrF₂NO₃); MS (ESI): 592.2 (MH⁺).

### b) 1-[4-(4-{1-(4-Fluor-phenyl)-3-[3-(4-fluor-phenyl)-3-hydroxy-propyl]-4-oxo-azetidin-2- yl}-phenoxymethyl)-benzyl]-4-aza-1-azonia-bicyclo[2.2.2]octan Bromid (13):

180 mg (12) werden mit 300 mg 1,4-Diaza-bicyclo[2.2.2]octan (DABCO) in 5ml Toluol gelöst und 90 min. bei 80°C gerührt. Nach Beendigung der Reaktion läst man das Gemisch abkühlen und der farblose Feststoff wird abgesaugt. Man erhält 195 mg Produkt (13) mit dem Molekulargewicht 704,66 (C₃₈H₄₀BrF₂N₃O₃); MS (ESI): 624.30 (MH⁺).

### Beispiel V

### 1-(4-Fluor-phenyl)-3-[3-(4-fluor-phenyl)-3-hydroxy-propyl]-4-(4-{4-[(2,3,4,5,6-pentahydroxy-hexylamin)-methyl]-benzyloxy}-phenyl)-azetidin-2-on (14)

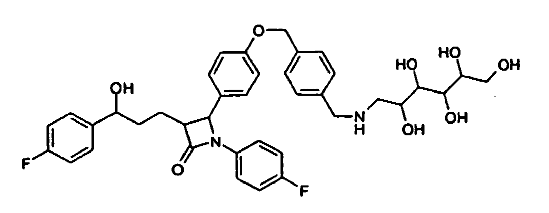

60 mg (12) werden mit 150 mg Glucamin in 5ml Dimethylformamid gelöst und 90 min. bei 80°C gerührt. Nach Beendigung der Reaktion wird das Gemisch in Essigsäureethylester gelöst und zweimal mit Wasser ausgeschüttelt. Die organische Phase wird über Magnesiumsulfat getrocknet, filtriert und im Vakuum eingeengt. Der Rückstand wird mit Flashchromatographie (Methylenchlorid/Methanol/conc. Ammoniak 30/10/3) gereinigt. Ausbeute 33 mg farbloser Feststoff (14) mit dem Molekulargewicht 692,76 (C₃₈H₄₂F₂N₂O₈); MS (ESI): 693,5 (MH⁺).

### Beispiel VI

### 1-[2-(4-{1-(4-Fluor-phenyl)-3-[3-(4-fluor-phenyl)-3-hydroxy-propyl]-4-oxo-azetidin-2- yl}-phenoxymethyl)-benzyl]-4-aza-1-azonia-bicyclo[2.2.2]octan Bromid (16):

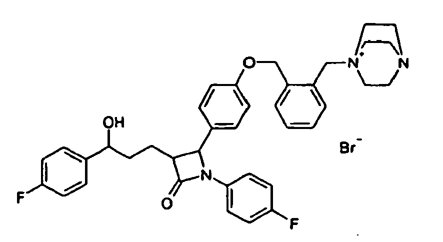

### a) 4-[4-(2-Brommethyl-benzyloxy)-phenyl]-1-(4-fluor-phenyl)-3-[3-(4-fluor-phenyl)-3- hydroxy-propyl]-azetidin-2-on (15):

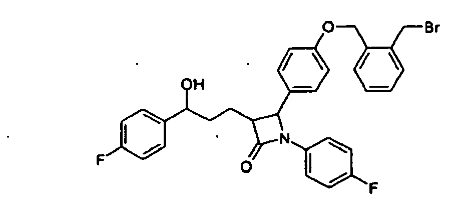

1-(4-Fluor-phenyl)-3-[3-(4-fluor-phenyl)-3-hydroxy-propyl]-4-(4-hydroxy-phenyl)-azetidin-2-on (11) wird mit 1,2-Bis-brommethyl-benzol und Kaliumcarbonat analog Beispiel IV umgesetzt und man erhält einen farbosen Feststoff (15) mit dem Molekulargewicht 592,49 (C₃₂H₂₈BrF₂NO₃); MS (ESI): 592.2 (MH⁺).

### b) 1-[4-(4-{1-(4-Fluor-phenyl)-3-[3-(4-fluor-phenyl)-3-hydroxy-propyl]-4-oxo-azetidin-2- yl}-phenoxymethyl)-benzyl]-4-aza-1-azonia-bicyclo[2.2.2]octan Bromid (16):

(15) wird mit DABCO in Toluol gelöst und analog Beispiel IV umgesetzt und man erhält das Produkt (16) als farblosen Feststoff mit dem Molekulargewicht 704,66 (C₃₈H₄₀BrF₂N₃O₃); MS (ESI): 624.30 (MH⁺).

### Beispiel VII

### 1-(4-Fluor-phenyl)-3-[3-(4-fluor-phenyl)-3-hydroxy-propyl]-4-(4-{2-[(2,3,4,5,6-pentahydroxy-hexylamin)-methyl]-benzyloxy}-phenyl)-azetidin-2-on (17):

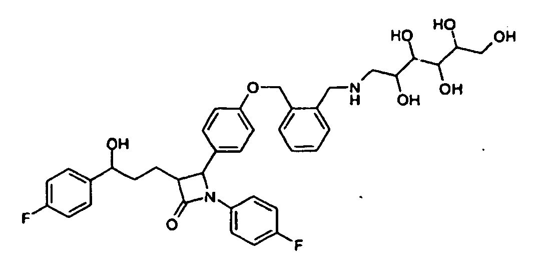

(15) wird mit Glucamin in Dimethylformamid gelöst und analog Beispiel V umgesetzt und man erhält das Produkt (17) als farblosen Feststoff mit dem Molekulargewicht 692,76 (C₃₈H₄₂F₂N₂O₈); MS (ESI): 693,5 (MH⁺).

### Beispiel VIII

### 1-[3-(4-{1-(4-Fluor-phenyl)-3-[3-(4-fluor-phenyl)-3-hydroxy-propyl]-4-oxo-azetidin-2- yl}-phenoxymethyl)-benzyl]-4-aza-1-azonia-bicyclo[2.2.2]octan Bromid (19):

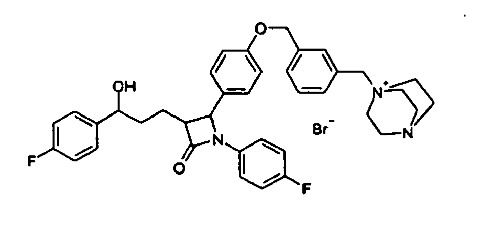

### a) 4-[4-(3-Brommethyl-benzyloxy)-phenyl]-1-(4-fluor-phenyl)-3-[3-(4-fluor-phenyl)-3- hydroxy-propyl]-azetidin-2-on (18):

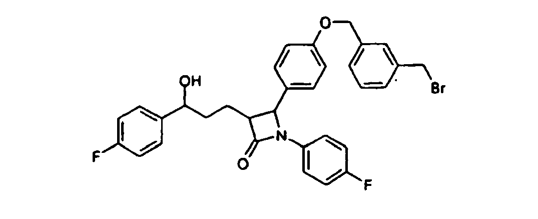

1-(4-Fluor-phenyl)-3-[3-(4-fluor-phenyl)-3-hydroxy-propyl]-4-(4-hydroxy-phenyl)-azetidin-2-on (11) wird mit 1,3-Bis-brommethyl-benzol und Kaliumcarbonat analog Beispiel IV umgesetzt und man erhält einen farbosen Feststoff (18) mit dem Molekulargewicht 592,49 (C₃₂H₂₈BrF₂NO₃); MS (ESI): 592.2 (MH⁺).

### b) 1-[3-(4-{1-(4-Fluor-phenyl)-3-[3-(4-fluor-phenyl)-3-hydroxy-propyl]-4-oxo-azetidin-2- yl}-phenoxymethyl)-benzyl]-4-aza-1-azonia-bicyclo[2.2.2]octan Bromid (19):

(18) wird mit DABCO in Toluol gelöst und analog Beispiel IV umgesetzt und man erhält das Produkt (19) als farblosen Feststoff mit dem Molekulargewicht 704,66 (C₃₈H₄₀BrF₂N₃O₃); MS (ESI): 624.30 (MH⁺).

### Beispiel IX

### 1-(4-Fluor-phenyl)-3-[3-(4-fluor-phenyl)-3-hydroxy-propyl]-4-(4-{3-[(2,3,4,5,6-pentahydroxy-hexylamin)-methyl]-benzyloxy}-phenyl)-azetidin-2-on (20):

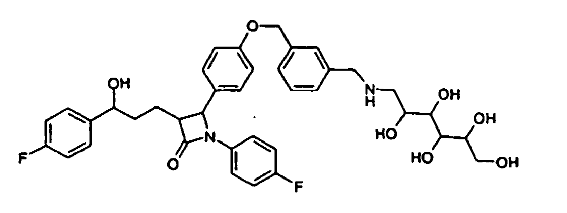

(18) wird mit Glucamin in Dimethylformamid gelöst und analog Beispiel V umgesetzt und man erhält das Produkt (20) als farblosen Feststoff mit dem Molekulargewicht 692,76 (C₃₈H₄₂F₂N₂O₈); MS (ESI): 693,5 (MH⁺).

### Beispiel X

### 1-[4'-(4-{1-(4-Fluor-phenyl)-3-[3-(4-fluor-phenyl)-3-hydroxy-propyl]-4-oxo-azetidin-2- yl}-phenoxymethyl)-biphenyl-4-ylmethyl]-4-aza-1-azonia-bicyclo[2.2.2]octan Bromid (22):

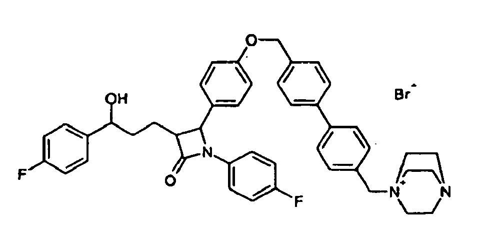

### a) 4-[4-(4'-Brommethyl-biphenyl-4-ylmethoxy)-phenyl]-1-(4-fluor-phenyl)-3-[3-(4-fluor- phenyl)-3-hydroxy-propyl]-azetidin-2-on (21):

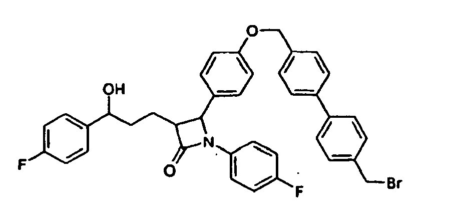

1-(4-Fluor-phenyl)-3-[3-(4-fluor-phenyl)-3-hydroxy-propyl]-4-(4-hydroxy-phenyl)-azetidin-2-on (11) wird mit 4,4'-Bis-brommethyl-biphenyl und Kaliumcarbonat analog Beispiel IV umgesetzt und man erhält einen farbosen Feststoff (21) mit dem Molekulargewicht 668,54 (C₃₈H₃₂BrF₂NO₃); MS (ESI): 668,1 (MH⁺).

### b) 1-[4'-(4-{1-(4-Fluor-phenyl)-3-[3-(4-fluor-phenyl)-3-hydroxy-propyl]-4-oxo-azetidin-2- yl}-phenoxymethyl)-biphenyl-4-ylmethyl]-4-aza-1-azonia-bicyclo[2.2.2]octan Bromid (22):

(21) wird mit DABCO in Toluol gelöst und analog Beispiel IV umgesetzt und man erhält das Produkt (22) als farblosen Feststoff mit dem Molekulargewicht 780,76 (C₄₄H₄₄BrF₂N₃O₃); MS (ESI): 700,3 (MH⁺).

### Beispiel XI

### 1-(4-Fluor-phenyl)-3-[3-(4-fluor-phenyl)-3-hydroxy-propyl]-4-(4-{4'-[(2,3.4,5,6-pentahydroxy-hexylamin)-methyl]-biphenyl-4-ylmethoxy}-phenyl)-azetidin-2-on (23):

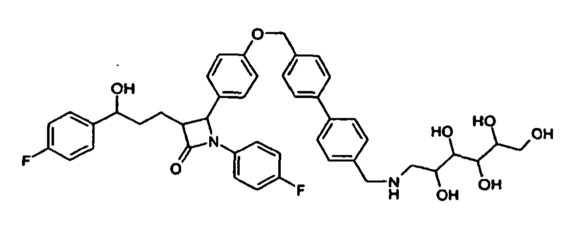

(21) wird mit Glucamin in Dimethylformamid gelöst und analog Beispiel V umgesetzt und man erhält das Produkt (23) als farblosen Feststoff mit dem Molekulargewicht 768,86 (C₄₄H₄₆F₂N₂O₈); MS (ESI): 769,3 (MH⁺).

### Beispiel XII

### 1-(4-{4-[3-[3-(4-Fluor-phenyl)-3-hydroxy-propyl]-2-(4-methoxy-phenyl)-4-oxo-azetidin-1- yl]-phenoxymethyl}-benzyl)-4-aza-1-azonia-bicyclo[2.2.2]octan Bromid (26):

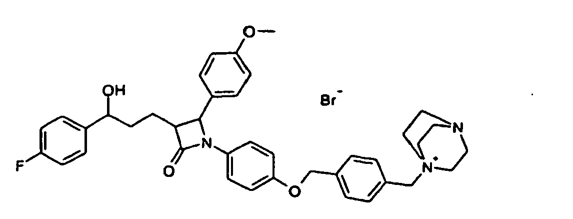

### a) 3-[3-(4-Fluor-phenyl)-3-hydroxy-propyl]-1-(4-hydroxy-phenyl)-4-(4-methoxy-phenyl)- azetidin-2-on (24):

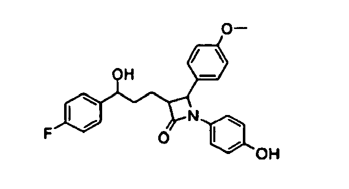

Diese Verbindung wird, wie vorn für 1-(4-Aminomethyl-phenyl)-3-[3-(4-fluor-phenyl)-3-hydroxy-propyl]-4-(4-methoxy-phenyl)- azetidin-2-on beschrieben, hergestellt (Stufe a, b, c, und d), mit dem Unterschied, dass 4-[(4-Methoxy-benzylidene)-amino]-phenol Einsatz finden. Man erhält 3-[3-(4-Fluor-phenyl)-3-hydroxy-propyl]-1-(4-hydroxy-phenyl)-4-(4-methoxy-phenyl)- azetidin-2-on (24) mit dem Molekulargewicht 421,47 (C₂₅H₂₄FNO₄); MS (ESI): 422,2 (MH⁺).

### b) 1-[4-(4-Brommethyl-benzyloxy)-phenyl]-3-[3-(4-fluor-phenyl)-3-hydroxypropyl]-4-(4- methoxy-phenyl)-azetidin-2-on (25):

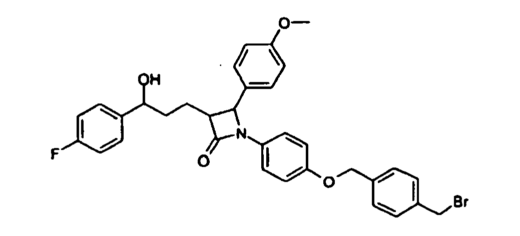

3-[3-(4-Fluor-phenyl)-3-hydroxy-propyl]-1-(4-hydroxy-phenyl)-4-(4-methoxy-phenyl)- azetidin-2-on wird mit 1,4-Bis-brommethyl-benzol und Kaliumcarbonat analog Beispiel IV umgesetzt und man erhält einen farbosen Feststoff (25) mit dem Molekulargewicht 604,52 (C₃₃H₃₁BrFNO₄); MS (ESI): 605,2 (MH⁺).

### c) 1-(4-{4-[3-[3-(4-Fluor-phenyl)-3-hydroxy-propyl]-2-(4-methoxy-phenyl)-4-oxo-azetidin-1- yl]-phenoxymethyl}-benzyl)-4-aza-1-azonia-bicyclo[2.2.2]octan Bromid (26):

(25) wird mit DABCO in Toluol gelöst und analog Beispiel IV umgesetzt und man erhält das Produkt (26) als farblosen Feststoff mit dem Molekulargewicht 716.70 (C₃₉H₄₃BrFN₃O₄); MS (ESI): 636.3 (MH⁺).

### Beispiel XIII

### 3-[3-(4-Fluor-phenyl)-3-hydroxy-propyl]-4-(4-methoxy-phenyl)-1-(4-{4-[(2,3,4,5,6-pentahydroxy-hexylamin)-methyl]-benzyloxy}-phenyl)-azetidin-2-on (27):

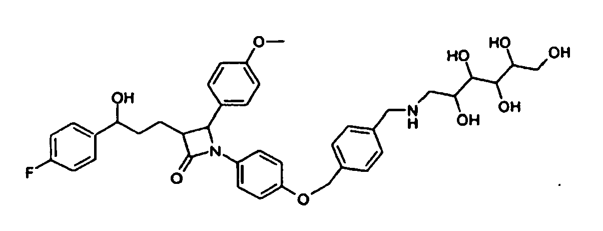

(25) wird mit Glucamin in Dimethylformamid gelöst und analog Beispiel V umgesetzt und man erhält das Produkt (27) als farblosen Feststoff mit dem Molekulargewicht 704.80 (C₃₉H₄₅FN₂O₉); MS (ESI): 705,31 (MH⁺).

### Beispiel XIV

### 1-[4-(4-{3-[1-(4-Fluor-phenyl)-2-(4-methoxy-phenyl)-4-oxo-azetidin-3-yl]-1-hydroxy- propyl}-phenoxymethyl)-benzyl]-4-aza-1-azonia-bicyclo[2.2.2]octan Bromid (30):

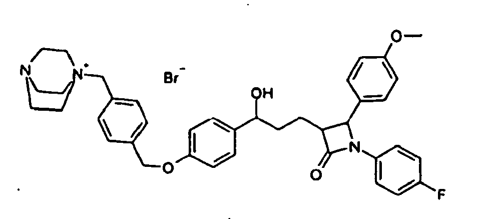

### a) 1-(4-Fluor-phenyl)-3-[3-hydroxy-3-(4-hydroxy-phenyl)-propyl]-4-(4-methoxy-phenyl)- azetidin-2-on (28):

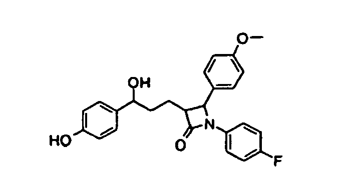

Diese Verbindung wird, wie vorn für 1-(4-Aminomethyl-phenyl)-3-[3-(4-fluor-phenyl)-3-hydroxy-propyl]-4-(4-methoxy-phenyl)- azetidin-2-on beschrieben, hergestellt, mit dem Unterschied, dass 3-[5-(4-Hydroxy-phenyl)-5-hydroxy-pentanoyl]-4-phenyl-oxazolidin-2-on und (4-Fluorphenyl)-(4-methoxy-benzylidene)-amin Einsatz finden. Man erhält 1-(4-Fluorphenyl)-3-[3-hydroxy-3-(4-hydroxy-phenyl)-propyl]-4-(4-methoxy-phenyl)- azetidin-2-on (28) mit dem Molekulargewicht 421,47 (C₂₅H₂₄FNO₄); MS (ESI): 422,2 (MH⁺).

### b) 3-{3-[4-(4-Brommethyl-benzyloxy)-phenyl]-3-hydroxy-propyl}-1-(4-fluor-phenyl)-4-(4- methoxy-phenyl)-azetidin-2-on (29):

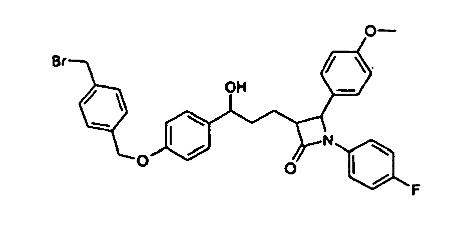

1-(4-Fluor-phenyl)-3-[3-hydroxy-3-(4-hydroxy-phenyl)-propyl]-4-(4-methoxy-phenyl)-azetidin-2-on wird mit 1,4-Bis-brommethyl-benzol und Kaliumcarbonat analog Beispiel IV umgesetzt und man erhält einen farbosen Feststoff (29) mit dem Molekulargewicht 604,52 (C₃₃H₃₁BrFNO₄); MS (ESI): 605,2 (MH⁺).

### c) 1-[4-(4-{3-[1-(4-Fluor-phenyl)-2-(4-methoxy-phenyl)-4-oxo-azetidin-3-yl]-1-hydroxy- propyl}-phenoxymethyl)-benzyl]-4-aza-1-azonia-bicyclo[2.2.2]octan Bromid (30):

(29) wird mit DABCO in Toluol gelöst und analog Beispiel IV umgesetzt und man erhält das Produkt (30) als farblosen Feststoff mit dem Molekulargewicht 716.70 (C₃₉H₄₃BrFN₃O₄); MS (ESI): 636.3 (MH⁺).

### Beispiel XV

### 1-{4-[(4-{1-(4-Fluor-phenyl)-3-[3-(4-fluor-phenyl)-3-hydroxy-propyl]-4-oxo-azetidin-2- yl}-benzylcarbamoyl)-methyl]-benzyl}-4-aza-1-azonia-bicyclo[2.2.2]octan; trifluor- acetat (35):

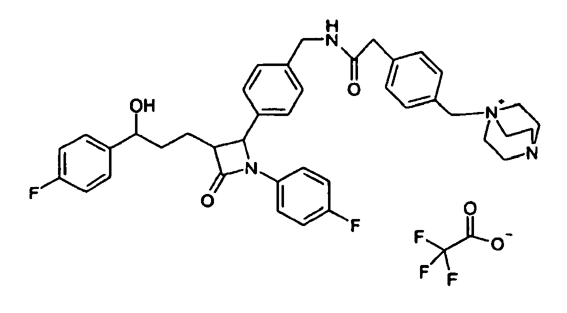

### a) 1-(4-Carboxymethyl-benzyl)-4-aza-1-azonia-bicyclo[2.2.2]octan; bromid (31):

Zu einer Lösung von 1.5 g 1,4-Diaza-bicyclo[2.2.2]octan in 10 ml Dimethylsulfoxid werden bei 70°C 1.0 g (3-Bromomethyl-phenyl)-essigsäure in 5 ml Dimethylsulfoxid gegeben. Nach 1 h werden 100 ml Wasser zugegeben und gefriergetrocknet. Der Rückstand wird mit Aceton digeriert. Der Rückstand enthält das Produkt mit einem Molekulargewicht von 261.35 (Kation: C₁₅H₂₁N₂O₂⁺); MS (ESI) 261.1 (M⁺).

### b) 4-[5-(4-Fluor-phenyl)-1-(4-fluor-phenylamino)-5-hydroxy-2-(2-oxo-4-phenyl-oxazolidin-3- carbonyl)-pentyl]-benzonitril (32):

2.5 g 3-[5-(4-Fluor-phenyl)-5-hydroxy-pentanoyl]-4-phenyl-oxazolidin-2-on werden in 30 ml Dichlormethan unter Argon gelöst, dazu gibt man 3.9 g 4-[(4-Fluor-phenylimino)-methyl]-benzonitril und kühlt auf -10°C. Zu dieser Mischung gibt man 6.4 ml Diisopropylethylamin und innerhalb von 30 min 4.05 ml Trimethylsilylchlorid, so dass die Temperatur -5°C nicht übersteigt. Bei dieser Temp. wird 1 Std. nachgerührt und dann auf -25°C gekühlt. Dann werden 0.8 ml Titantetrachlorid langsam zugegeben. Die dunkle Mischung wird über Nacht bei - 25 bis -30°C gerührt danach mit 35 ml 7proz. Weinsäurelösung zersetzt und 1 Std. bei Raumtemp. nachgerührt. Anschließend gibt man 15 ml einer 20proz. Natriumhydrogencarbonatlösung dazu und rührt erneut 1 Std. Nach Phasentrennung wird die org. Phase mit 30 ml Waser gewaschen, über Magnesiumsulfat getrocknet und auf ca. 10 ml eingeengt. Nach Zugabe von 2 ml Bistrimethylsilylacetamid erwärmt man 30 min. zum Rückfluss und engt danach i.Vak. ein. Der Rückstand wir d mit Ethylacetat/Heptan zur Kristallisation gebracht. Man saugt ab und trocknet i.Vak. Man erhält das Produkt mit dem Molekulargewicht 653.81 (C₃₇H₃₇F₂N₃O₄Si); MS (ESI+): 654.3 (M+H⁺), 582.2 (M+H⁺ -Si(CH₃)₃).

### c) {1-(4-Fluor-phenyl)-3-[3-(4-fluor-phenyl)-3-hydroxy-propyl]-4-oxo-azetidin-2-yl}- benzonitril (33):

2 g 4-[5-(4-Fluor-phenyl)-1-(4-fluor-phenylamino)-5-hydroxy-2-(2-oxo-4-phenyl-oxazolidin-3- carbonyl)-pentyl]-benzonitril werden in 20 ml Methyl-tert.-butyl-ether gelöst und mit 100 mg Tetrabutyl- ammoniumfluorid-Trihydrat und 1.3 ml Bistrimethylsilylacetamid ca. 1 h auf 40°C erwärmt. Man verfolgt die Reaktion im Dünnschichtchromatogramm. Nach beendeter Umsetzung setz man zunächst 0.2 ml Eisessig zu , rührt 30 min und engt ein. Der Rückstandwird mit 20ml einer Mischung von Isopropanol /2N Schwefelsäure = 10:1 versetzt und 1 Std. gerührt. Nach Zugabe einer Spatelspitze festem Natriumhydrogencarbonat engt man erneut i. Vak. ein, nimmt mit Ethylacetat auf, wäscht die org. Phase mit Wasser , trocknet und reinigt nach Entfernen des Lösemittels den Rückstand durch Säulenchromatographie (SiO₂, CH₂Cl₂/Methanol = 100: 1). Man erhält das Produkt mit dem Molekulargewicht 418.45 (C₂₅H₂₀F₂N₂O₂); MS (DCI+): 419 (M+H⁺).

### d) 4-(4-Aminomethyl-phenyl)-1-(4-fluor-phenyl)-3-[3-(4-fluor-phenyl)-3-hydroxy-propyl] azetidein-2 on (34):

200 mg {1-(4-Fluor-phenyl)-3-[3-(4-fluor-phenyl)-3-hydroxy-propyl]-4-oxo-azetidin-2-yl}- benzonitril werden in 20 ml Ethanol gelöst und mit 0.5 ml konz. Ammoniak über Raney-Nickel 30 Std bei 75 bar Wasserstoff und 25°C hydriert. Man saugt vom Katalysator ab, engt i. Vak. ein und reinigt den Rückstand durch Säulenfiltration (SiO₂, CH₂Cl₂/Methanol/. NH₃ conc = 100:10:1). Man erhält das Produkt mit dem Molekulargewicht 422.5 (C₂₅H₂₂F₂N₂O₂): MS (DCI+): 423 (M+H⁺), 405 (M+H⁺ - H₂O).

### e) 1-{4-[(4-{1-(4-Fluor-phenyl)-3-[3-(4-fluor-phenyl)-3-hydroxy-propyl]-4-oxo-azetidin-2- yl}-benzylcarbamoyl)-methyl]-benzyl}-4-aza-1-azonia-bicyclo[2.2.2]octan; trifluoracetat (35):

Zu einer Lösung aus 84 mg 1-(4-Carboxymethyl-benzyl)-4-aza-1-azonia-bicyclo[2.2.2]octan; bromid, 64 µl Diisopropylcarbodiimid, 56 mg Hydroxybenzotriazol in 2 ml Dimethylformamid wird eine Lösung aus 70 mg 4-(4-Aminomethyl-phenyl)-1-(4-fluor-phenyl)-3-[3-(4-fluor-phenyl)-3-hydroxy-propyl]-azetidin-2-on, 23 µl Triethylamin in 0.5 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1 % Trifluoressigsäure) = 80/20 -> 10/90) getrennt. Man erhält das Produkt mit einem Molekulargewicht von 665.81 (Kation: C₄₀H₄₃F₂N₄O₃); MS (ESI) 665.33 (M⁺).

### Beispiel XVI

### 1-{4-[(4-{1-(4-Fluor-phenyl)-3-[3-(4-fluor-phenyl)-3-hydroxy-propyl]-4-oxo-azetidin-2- yl}-benzylcarbamoyl)-methyl]-benzyl}-1-azonia-bicyclo[2.2.2]octan; trifluoracetat (37):

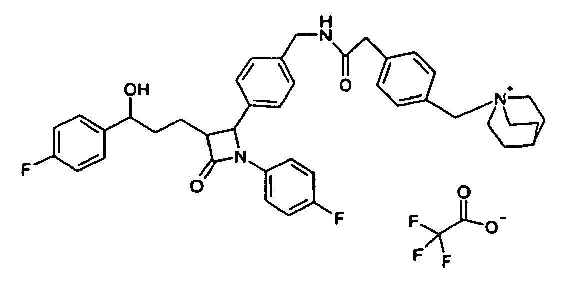

### a) 1-(4-Carboxymethyl-benzyl)-1-azonia-bicyclo[2.2.2]octan; bromid (36):

Die Synthese erfolgt analog Beispiel XVa) ausgehend von 1.67 g 1-Aza-bicyclu[2.2.2]octan. Man erhält das Produkt mit einem Molekulargewicht von 260.36 (Kation: C₁₆H₂₂N₁O₂⁺); MS (ESI) 260.1 (M⁺).

### b) 1-{4-[(4-{1-(4-Fluoro-phenyl)-3-[3-(4-fluoro-phenyl)-3-hydroxy-propyl]-4-oxo-azetidin-2-yl}-benzylcarbamoyl)methyl]-benzyl}-1-azonia-bicyclo[2.2.2]octane; trifluoracetat (37):

Die Synthese erfolgt analog Beispiel XVe) ausgehend 70 mg 4-(4-Aminomethylphenyl)-1-(4-fluor-phenyl)-3-[3-(4-fluor-phenyl)-3-hydroxy-propyl]-azetidin-2-on und 85 mg 1-(4-Carboxymethyl-benzyl)-1-azonia-bicyclo[2.2.2]octan; bromid. Man erhält das Produkt mit einem Molekulargewicht von 664.82 (Kation: C₄₁H₄₄F₂N₃O₃⁺); MS (ESI) 664.33 (M⁺).

### Beispiel XVII

### 1-{4-[(4-{1-(4-Fluor-phenyl)-3-[3-(4-fluor-phenyl)-3-hydroxy-propyl]-4-oxo-azetidin-2- yl}-benzylcarbamoyl)-methyl]-benzyl}-1,4-dimethyl-piperazin-1-ium; trifluoracetat (39):

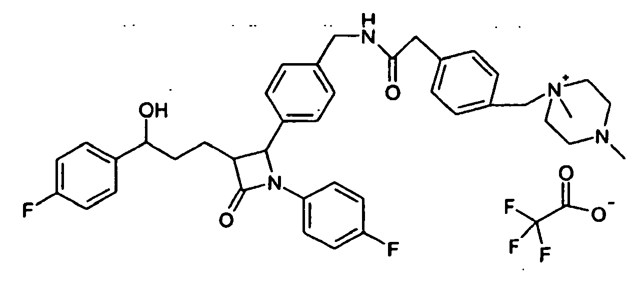

### a) 1-(4-Carboxymethyl-benzyl)-1,4-dimethyl-piperazin-1-ium; bromid (38):

Die Synthese erfolgt analog Beispiel XVa) ausgehend von 1.02 ml 1,4-Dimethylpiperazin. Man erhält das Produkt mit einem Molekulargewicht von 263.36 (Kation: C₁₅H₂₃N₂O₂⁺); MS (ESI) 263.1 (M⁺).

### b) 1-{4-[(4-{1-(4-Fluor-phenyl)-3-[3-(4-fluor-phenyl)-3-hydroxy-propyl]-4-oxo-azetidin-2- yl}-benzylcarbamoyl)-methyl]-benzyl}-1,4-dimethyl-piperazin-1-ium; trifluoracetat (39):

Die Synthese erfolgt analog Beispiel XVe) ausgehend 70 mg 4-(4-Aminomethylphenyl)-1-(4-fluor-phenyl)-3-[3-(4-fluor-phenyl)-3-hydroxy-propyl]-azetidin-2-on und 86 mg 1-(4-Carboxymethyl-benzyl)-1,4-dimethyl-piperazin-1-ium; bromid. Man erhält das Produkt mit einem Molekulargewicht von 667.82 (Kation: C₄₀H₄₅F₂N₄O₃⁺); MS (ESI) 667.34 (M⁺).

### Beispiel XVIII

### 4-{4-[(4-{1-(4-Fluor-phenyl)-3-[3-(4-fluor-phenyl)-3-hydroxy-propyl]-4-oxo-azetidin-2-yl}-benzylcarbamoyl)methyl]-benzyl}-4-methyl-morpholin-4-ium; trifluoracetat (41):

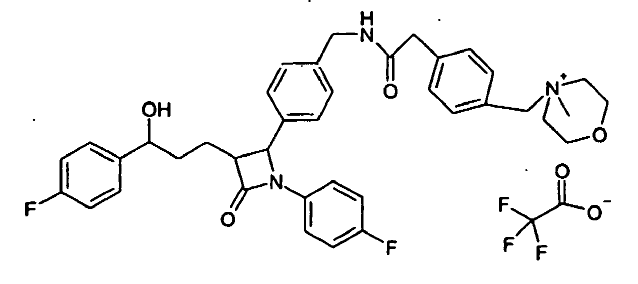

### a) 4-(4-Carboxymethyl-benzyl)-4-methyl-morpholin-4-ium; bromid (40):

Die Synthese erfolgt analog Beispiel XVa) ausgehend von 1.65 ml N-Methylmorpholin. Man erhält das Produkt mit einem Molekulargewicht von 250.32 (Kation: C₁₄H₂₀N₁D₃⁺); MS (ESI) 250.1 (M⁺).

### b) 4-{4-[(4-{1-(4-Fluor-phenyl)-3-[3-(4-fluor-phenyl)-3-hydroxy-propyl]-4-oxo-azetidin-2-yl}-benzylcarbamoyl)-methyl]-benzyl}-4-methyl-morpholin-4-ium; trifluoracetat (41):

Synthese erfolgt analog Beispiel XVe) ausgehend 70 mg 4-(4-Aminomethylphenyl)-1-(4-fluor-phenyl)-3-[3-(4-fluor-phenyl)-3-hydroxy-propyl]- azetidin-2-on und 82 mg 4-(4-Carboxymethyl-benzyl)-4-methyl-morpholin-4-ium; bromid. Man erhält das Produkt mit einem Molekulargewicht von 654.78 (Kation: C₃₉H₄₂F₂N₃O₄⁺); MS (ESI) 654.31 (M⁺).

### Beispiel XIX

### 1-{4-[(4-{1-(4-Fluor-phenyl)-3-[3-(4-fluor-phenyl)-3-hydroxy-propyl]-4-oxo-azetidin-2-yl}-benzylcarbamoyl)-methyl]-benzyl}-1,4,7-trimethyl-[1,4,7]triazonan-1-ium; trifluoracetat (43):

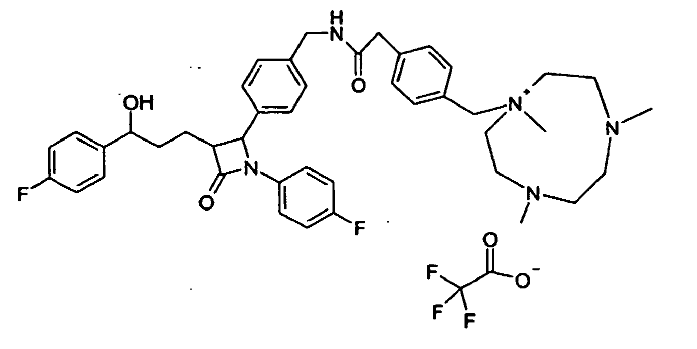

### a) 1,4,7-Trimethyl-1-[4-(2-oxo-propyl)-benzyl]-[1,4,7]triazonan-1-ium; bromid (42):

Die Synthese erfolgt analog Beispiel XVa) ausgehend von 500 mg 1,4,7-Trimethyl-[1,4,7]triazonan. Man erhält das Produkt mit einem Molekulargewicht von 321.47 (Kation: C₁₈H₃₁N₃O₂⁺); MS (ESI) 321.2 (M⁺).

### b) 1-{4-[(4-{1-(4-Fluor-phenyl)-3-[3-(4-fluor-phenyl)-3-hydroxy-propyl]-4-oxo-azetidin-2- yl}-benzylcarbamoyl)-methyl]-benzyl}-1,4,7-trimethyl[1,4,7]triazonan-1-ium; trifluoracetat (43):

Die Synthese erfolgt analog Beispiel XVe) ausgehend 70 mg 4-(4-Aminomethylphenyl)-1-(4-fluor-phenyl)-3-[3-(4-fluor-phenyl)-3-hydroxy-propyl]- azetidin-2-on und 200 mg 1,4,7-Trimethyl-1-[4-(2-oxo-propyl)benzyl]-[1,4,7]triazonan-1-ium; bromid. Man erhält das Produkt mit einem Molekulargewicht von 724.92 (Kation: C₄₀H₄₅F₂N₄O₃⁺); MS (ESI) 724.40 (M⁺).

### Beispiel XX

### 1-[4-({4-[3-(3-Hydroxy-3-phenyl-propyl)-2-oxo-4-phenyl-azetidin-1-yl]-benzylcarbamoyl}- methyl)-benzyl]-4-aza-1-azonia-bicyclo[2.2.2]octan; triflucracotat (44):

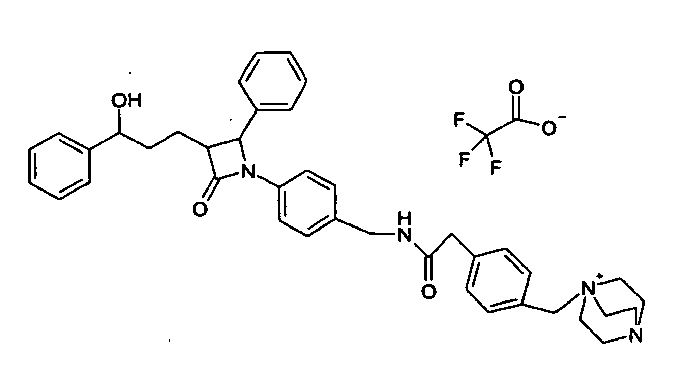

Die Synthese erfolgt analog Beispiel XVe) ausgehend 60 mg 1-(4-Aminomethylphenyl)-3-(3-hydroxy-3-phenyl-propyl)-4-phenyl-azetidin-2-on (8) und 82 mg 1-(4-Carboxymethyl-benzyl)-4-aza-1-azonia-bicyclo[2.2.2]octan; bromid. Man erhält das Produkt mit einem Molekulargewicht von 629.83 (Kation: C₄₀H₄₅F₂N₄O₃⁺); MS (ESI) 629.35 (M⁺).

### Beispiel XXI

N-(4-{1-(4-Fluor-phenyl)-3-[3-(4-fluor-phenyl)-3-hydroxy-propyl]-4-oxo-azetidin-2-yl}-benzyl)-2-{4-[(2,3,4,5,6-pentahydroxy-hexylamino)-methyl]-phenyl}-acetamid (46):

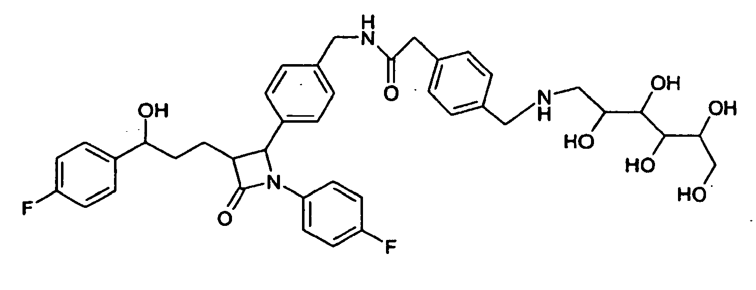

### a) {4-[(2,3,4,5,6-Pentahydroxy-hexylamino)-methyl]-phenyl}-essigsäure (45):

Die Synthese erfolgt analog Beispiel XVa) ausgehend von 989 mg 6-Aminohexan-1,2,3,4,5-pentaol. Man erhält das Produkt mit einem Molekulargewicht von 329.35 (C₁₅H₂₃N₁O₇); MS (ESI) 330.2 (M + H⁺).

### b) N-(4-{1-(4-Fluor-phenyl)-3-[3-(4-fluor-phenyl)-3-hydroxy-propyl]-4-oxo-azetidin-2-yl}-benzyl)-2-{4-[(2,3,4,5,6-pentahydroxy-hexylamino)-methyl]-phenyl}-acetamid (46):

Die Synthese erfolgt analog Beispiel XVe) ausgehend 70 mg 4-(4-Aminomethylphenyl)-1-(4-fluor-phenyl)-3-[3-(4-fluor-phenyl)-3-hydroxy-propyl]-azetidin-2-on und 110 mg {4-[(2,3,4,5,6-Pentahydroxy-hexylamino)-methyl]-phenyl}-essigsäure. Man erhält das Produkt mit einem Molekulargewicht von 733.82 (C₄₀H₄₅F₂N₃O₈⁺); MS (ESI) 734.32 (M + H⁺).

### Beispiel XXII

### N-(4-{1-(4-Fluor-phenyl)-3-[3-(4-fluor-phenyl)-3-hydroxy-propyl]-4-oxo-azetidin-2-yl}-benzyl)-2-(4-{[methyl-(2,3,4,5,6-pentahydroxy-hexyl)-amino]-methyl}-phenyl)-acetamid (48):

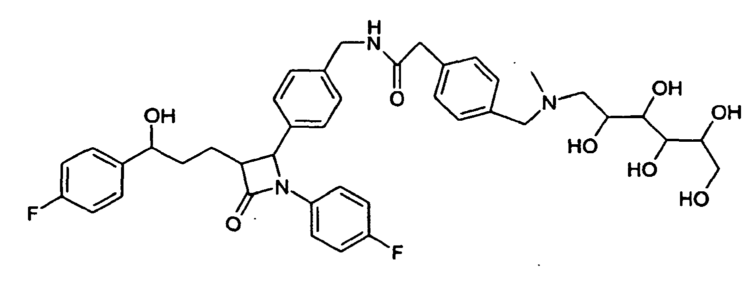

### a) (4-{[Methyl-(2,3,4,5,6-pentahydroxy-hexyl)-amino]-methyl}-phenyl)-essigsäure (47):

Die Synthese erfolgt analog Beispiel XVa) ausgehend von 1.06 g 6-Methylaminohexan-1,2,3,4,5-pentaol. Man erhält das Produkt mit einem Molekulargewicht von 343.38 (C₁₆H₂₅N₁O₇); MS (ESI) 344.2 (M + H⁺).

### b) N-(4-{1-(4-Fluor-phenyl)-3-[3-(4-fluor-phenyl)-3-hydroxy-propyl]-4-oxo-azetidin-2-yl}-benzyl)-2-(4-{[methyl-(2,3,4,5,6-pentahydroxy-hexyl)-amino]-methyl}-phenyl)-acetamid (48):

Die Synthese erfolgt analog Beispiel XVe) ausgehend 70 mg 4-(4-Aminomethylphenyl)-1-(4-fluor-phenyl)-3-[3-(4-fluor-phenyl)-3-hydroxy-propyl]- azetidin-2-on und 114 mg (4-{[Methyl-(2,3,4,5,6-pentahydroxy-hexyl)-amino]-methyl}-phenyl)-essigsäure. Man erhält das Produkt mit einem Molekulargewicht von 747.84 (C₄₁H₄₇F₂N₃O₈⁺); MS (ESI) 748.35 (M + H⁺).

**Tabelle 1: Verbindungen der Formel I**

| | | | | | | |
|---|---|---|---|---|---|---|
| 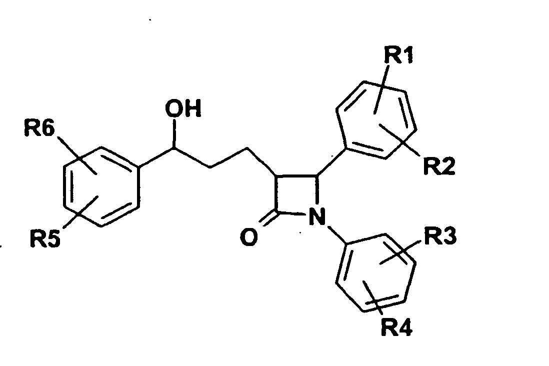 | | | | | | |

| **Bsp.** | **R1, R2** | **R3, R4** | **R5, R6** | **Salz** | **Molekulargewicht der freien Base bzw. Säure (berechnet)** | **Molekulargewicht (gefunden)** |
|---|---|---|---|---|---|---|
| XXIII | 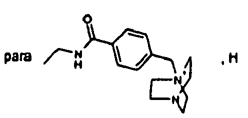 | para-F, H | para-F, H | Cl⁻ | 651,78 | 651,31 (M⁺) |
| XXIV | 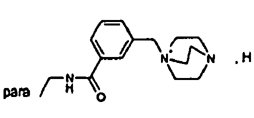 | para-F, H | para-F, H | CF₃COO⁻ | 651,78 | 651,31 (M⁺) |
| XXV | 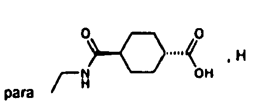 | para-F, H | para-F, H | - | 567,64 | 577,25 (MH⁺) |
| XXVI | 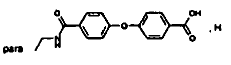 | para-F, H | para-F, H | - | 662,69 | 663,22 (MH⁺) |
| XXVII | para-O-CH₃, H | 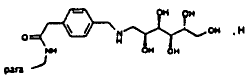 | para-F, H | CF₃COOH | 745,85 | 746,34 (MH⁺) |
| XXVIII | para-O-CH₃, H | 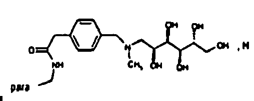 | para-F, H | CF₃COOH | 759,88 | 760,35 (MH⁺) |
| XXIX | 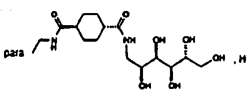 | para-F, H | para-F, H | - | 739,82 | 740,33 (MH⁺) |
| XXX | 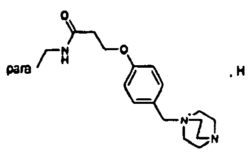 | para-F, H | para-F, H | CF₃COO⁻ | 695,84 | 659,34 (M⁺) |
| XXXI | 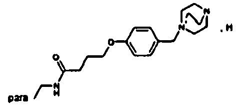 | para-F, H | para-F, H | CF₃COO⁻ | 709,86 | 709,35 (M⁺) |
| XXXII | 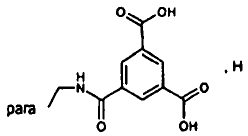 | para-F, H | para-F, H | - | 614,60 | 597,18 (MH⁺-H₂O) |
| XXXIII | 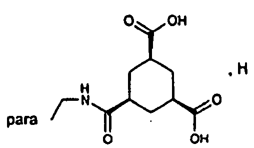 | para-F, H | para-F, H | - | 620,65 | 621,24 (MH⁺) |
| XXXIV | 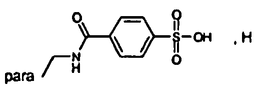 | para-F, H | para-F, H | - | 606,65 | 607,32 (MH⁺) |
| XXXV | 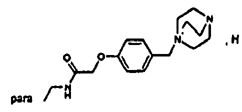 | para-F, H | para-F, H | CF₃COO⁻ | 681,33 | 681,5 (M⁺) |
| XXXVI | para-O-CH₃, H | 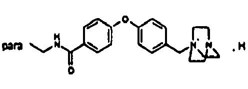 | para-F, H | Cl⁻ | 755,92 | 755,36 (M⁺) |
| XXXVII | para-O-CH₃, H | 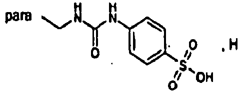 | H, H | - | 615,71 | 616,21 (MH⁺) |
| XXXVIII | 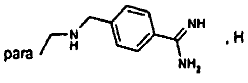 | para-F, H | para-F, H | CF₃COOH | 554,65 | 555,20 (MH⁺) |
| XXXIX | 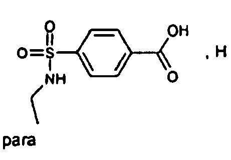 | para-F, H | para-F, H | - | 606,65 | 607,39 (MH⁺) |
| XL | para-O-CH₃, H | 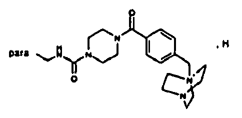 | para-F, H | Cl⁻ | 775,95 | 775,40 (M⁺) |
| XLI | para-O-CH₃, H | 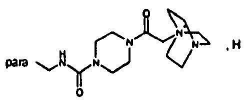 | para-F, H | Cl⁻ | 699,85 | 699,33 (M⁺) |
| XLII | para-O-CH₃, H | 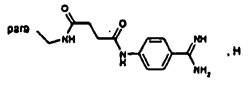 | para-F, H | HCl | 651,74 | 652,38 (MH⁺) |
| XLIII | para-O-CH₃, H | 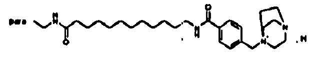 | para-F, H | Br⁻ | 860,51 | 860,6 (M⁺) |
| XLIV | para-O-CH₃, H | 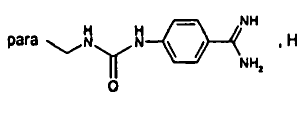 | para-F, H | - | 595,67 | 596,38 (MH⁺) |
| XLV | para-O-CH₃, H | 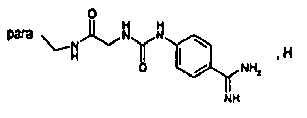 | para-F, H | CF₃COOH | 652,73 | 653,37 (MH⁺) |
| XLVI | para-O-CH₃, H | 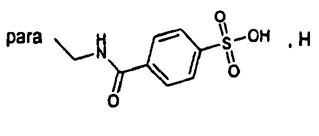 | para-F, H | - | 618,68 | 617,33 (M-H⁺; gemessen im Negativ-modus) |
| XLVII | para-O-CH₃, H | 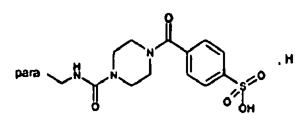 | para-F, H | - | 730,81 | 731,41 (MH⁺) |
| XLVIII | para-O-CH₃, H | 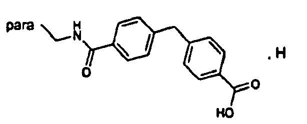 | para-F, H | - | 672,76 | 655,28 (MH⁺ -H₂O) |
| IL | para-O-CH₃, H | 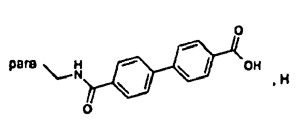 | para-F, H | - | 658,73 | 659,27 (MH⁺) |
| L | para-O-CH₃, H | 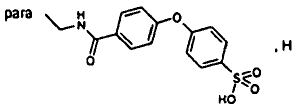 | para-F, H | - | 710,78 | 693,25 (MH⁺ -H₂O) |
| LI | para-O-CH₃, H | 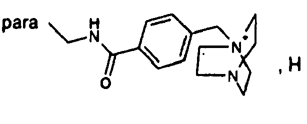 | para-F, H | Cl⁻ | 663,82 | 663,28 (M⁺) |
| LII | para-O-CH₃, H | 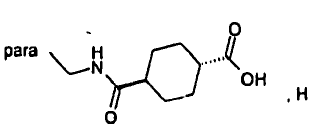 | para-F, H | - | 588.68 | 589,34 (MH⁺) |
| LIII | para-O-CH₃, H | 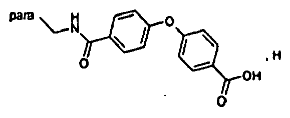 | para-F, H | - | 674.73 | 657,35 (MH⁺ - H₂O) |
| LIV | para-O-CH₃, H | 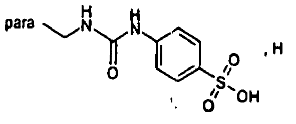 | para-F, H | - | 633,70 | 615,70 (M⁺ - H₂O) |
| LV | para-O-CH₃, H | 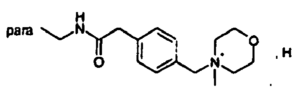 | para-F, H | Br | 666.72 | 666.33 (M⁺) |
| LVI | 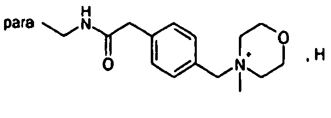 | para-F, H | para-F, H | Br | 654.68 | 654.31 (M⁺) |
| LVII | 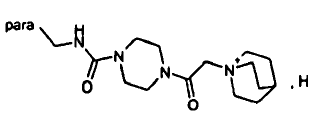 | para-F, H | para-F, H | I⁻ | 687,35 | 687,4 (M⁺) |
| LVIII | 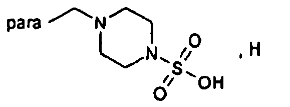 | para-F, H | para-F, H | - | 571,65 | 572,4 (MH⁺) |
| LIX | 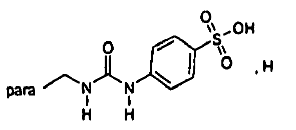 | para-F, H | para-F, H | - | 621,66 | 622,33 (MH⁺) |
| LX | para-O-CH₃, H | 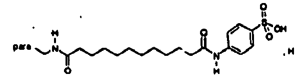 | para-F, H | - | 801,98 | 784,21 (M⁺ - H₂O) |
| LXI | para-O-CH₃, H | 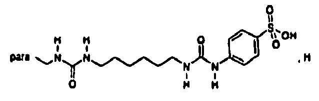 | para-F, H | - | 775,90 | 758,18 (M⁺ - H₂O) |
| LXII | para-O-CH₃, H | 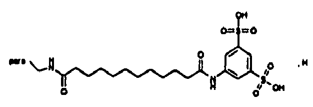 | para-F, H | - | 882.04 | 883,49 (MH+) |

Die erfindungsgemäßen Verbindungen der Formel I wurden mit der nachfolgend beschriebenen Methode auf ihre Wirkung geprüft:

### Beeinflussung der Cholesterolabsorption + ³H-Taurocholsäureausscheidung anhand der fäkalen Ausscheidung an der Maus, Ratte oder Hamster

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit (¹⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 ml/Maus Intralipid ® 20 (Pharmacia-Upjohn) ((Spikung mit 0,25 µCi ¹⁴C-Cholesterol in 0,1 mg Cholesterol) werden peroral mit der Schlundsonde verabreicht.

Testsubstanzen werden getrennt in 0,5 %/ (Methylcellulose (Sigma)/5% Solutol (BASF, Ludwigshafen ) oder geeignetem Vehikel angesetzt. Das Applikationsvolumen der Testsubstanz beträgt 0,5 ml /Maus. Die Testsubstanz wird unmittelbar vor der Testmahlzeit (Intralipid mit ¹⁴C-Cholesterol-labei) (Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C- Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H2O und ¹⁴C- CO2 wird hochgerechnet auf die ausgeschiedene Menge an ³H-Taurocholsäure bzw. ¹⁴C-Cholesterol (Dual-Isotopen-Technik ). Die ED₂₀₀-Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C-Cholesterol in die Leber halbiert (50%), bezogen auf eine Kontrollgruppe

Die folgenden ED₅₀-Werte belegen die Aktivität der erfindungsgemäßen Verbindungen der Formel I

| Beispiel Nr. | ED₅₀ (Leber) [mg/Maus] |
|---|---|
| I | < 0.1 |
| II | < 1.0 |
| IV | < 0.1 |
| V | < 0.1 |
| VI | 0.3 |
| VII | < 1.0 |
| VIII | < 1.0 |
| IX | < 0.1 |
| XV | < 1.0 |
| XXIII | 0.3 |
| XXV | 0.3 |
| XXVI | 0.1 |
| XXVII | 0.3 |
| XXIX | 0.3 |
| XXXI | 0.3 |
| XXXVI | 0.03 |
| XXXVII | 0.1 |
| XXXVIII | 0.1 |
| XLI | 0.03 |
| XLIII | 0.3 |
| XLIV | 0.3 |
| XLVI | 0.3 |
| XLVIII | 0.03 |
| L | 0.1 |
| LII | 0.3 |
| LIII | 0.03 |

Aus der Tabelle ist abzulesen, daß die Verbindungen der Formel I eine sehr gute Cholesterin senkende Wirkung besitzen.

### Resorbierbarkeit:

Die Resorbierbarkeit der Verbindungen der Formel I wurde Caco-Zellmodell geprüft (A.R. Hilgers et al., Caco-2 cell monolayers as a model for drug transport across the intestinal mucosa, Pharm. Res. 1990 , 7, 902).

Aus den Meßdaten ist abzulesen, daß die erfindungsgemäßen Verbindungen der Formel I gegenüber den im Stand der Technik beschriebenen Verbindungen (Referenzstruktur) eine deutlich geringere Resorption aufweisen.

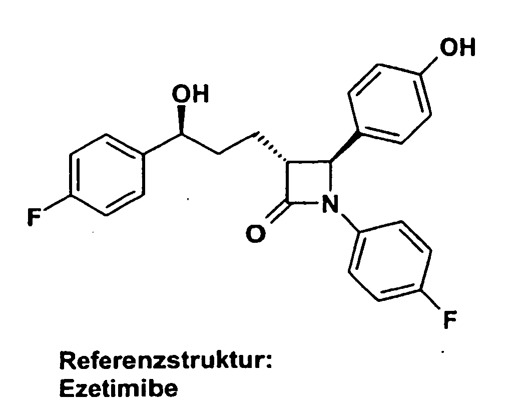

## Patentansprüche

1. Verbindungen der Formel I, 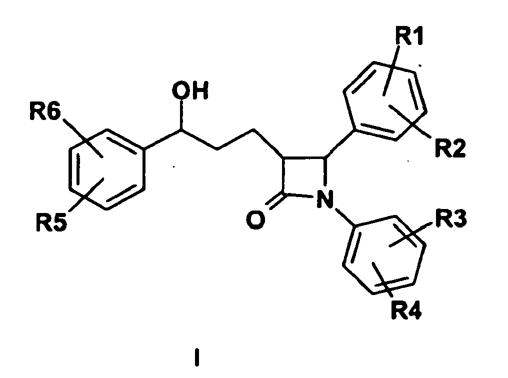 worin bedeuten
R2, R4, R5, R6 unabhängig voneinander H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)- Alkinyl, O-(C₁-C₆)-Alkyl, wobei in den Alkylresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂ , S-(C₁-C₆)- Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂. CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁- C₆)-Alkyl, CONH₂;
R1, R3 unabhängig voneinander -(CH₂)₀₋₁-NH-(C=O)₀₋₁-(C₀-C₂₅)- Alkylen-(C=O)₀₋₁-N(R13)₀₋₁(LAG) oder -(CH₂)₀₋₁-(C=O)₀₋₁-NH- (C₀-C₂₅)-Alkylen-(C=O)₀₋₁-N(R13)₀₋₁-(LAG) hat; worin ein oder mehrere C-Atome des Alkylenrests, durch bis zu dreifach mit R7 substituierte Aryl- oder Heteroarylreste oder durch bis zu dreifach mit R7substituierte (C₃-C₁₀)-Cycloalkyl- oder Heterocycloalkylreste ersetzt sind und worin ein oder mehrere C-Atome des Alkylenrests durch O-Atome ersetzt sein können;
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂. (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂NH₂, SO₂NH(C₁-C₆)-Alkyl, SO₂N[(C₁-C₆)-Alkyl]₂ , S-(C₁-C₆)- Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁- C₆)-Alkyl, CONH₂;
R7 F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂. (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
PO₃H₂, SO₃H, SO₂-NH₂, SO₂NH(C₁-C₆)-Alkyl, SO₂N[(C₁-C₆)- Alkyl]₂ , S-(C₁-C₆)-Alkyl, S-(CH₂)ₙ-Phenyl, SO-(C₁-C₆)-Alkyl, SO- (CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁- C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)- Alkyl, NH₂, NH(C₁-C₆)-Alkyl, N((C₁-C₆)-Alkyl)₂, SO₂-CH₃, COOH, COO-(C₁-C₆)-Alkyl, CONH₂;
LAG Monozuckerest, ein acyclischer, mono-, bi- oder tricyclischer Trialkylammoniumalkyl-Rest, eine Sulfonsäure oder eine Carbonsäure,
R13 H oder CH₃;
und wobei immer mindestens einer der Reste R1 oder R3 die Bedeutung -(CH₂)₀₋₁-NH-(C=O)₀₋₁-(C₀-C₂₅)-Alkylen-(C=O)₀₋₁-N(R13)₀₋₁-(LAG) oder - (CH₂)₀₋₁-(C=O)₀₋₁-NH-(C₀-C₂₅)-Alkylen-(C=O)₀₋₁-N(R13)₀₋₁-(LAG) hat und ein oder mehrere C-Atome des Alkylenrests durch bis zu dreifach substituierte Aryl- oder Heteroarylreste oder durch bis zu dreifach substituierte (C₃-C₁₀)-Cycloalkyl- oder Heterocycloalkylreste ersetzt sind und zusätzlich durch - S(O)ₙ-, mit n = 0 - 2, -O-, -(C=O)-, -N(CH₃)-, -N(Phenyl)- oder -NH- ersetzt sein können,
besitzen muß;
sowie deren physiologisch verträglichen Salze.

2. Verbindungen der Formeln I bis XXII

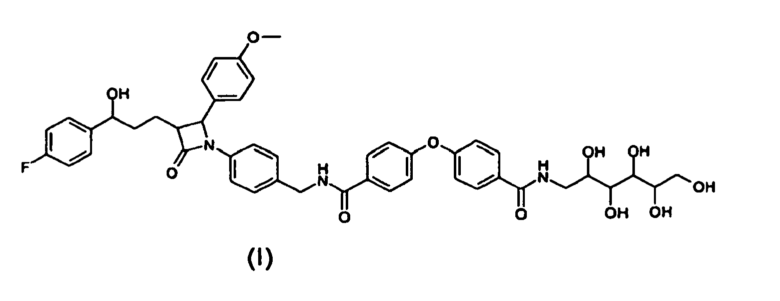

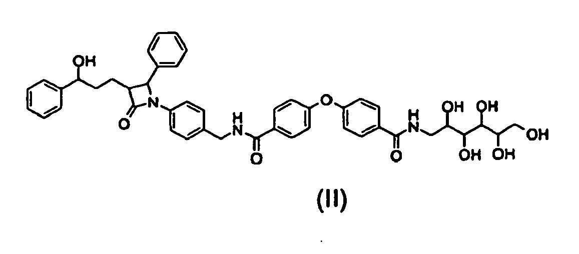

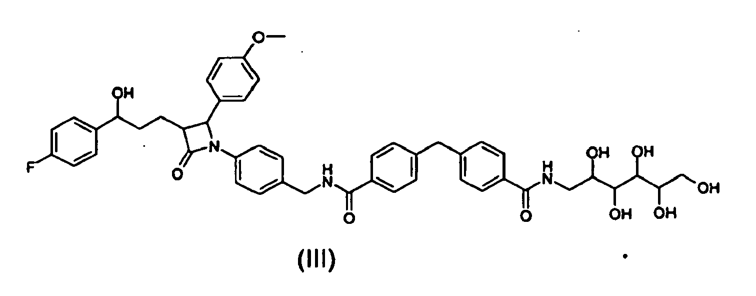

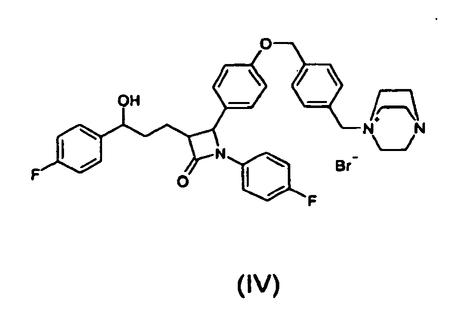

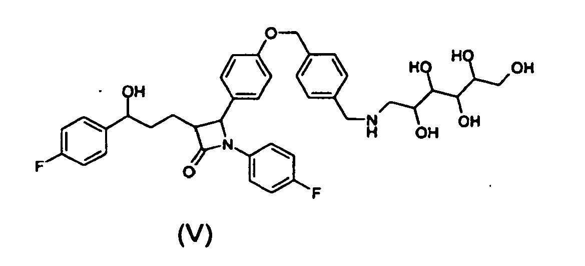

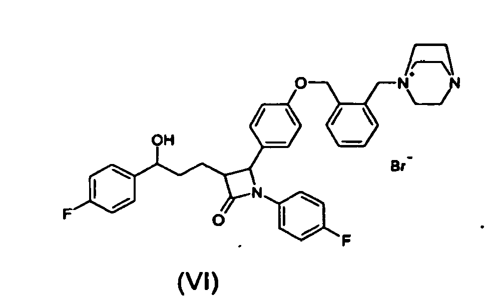

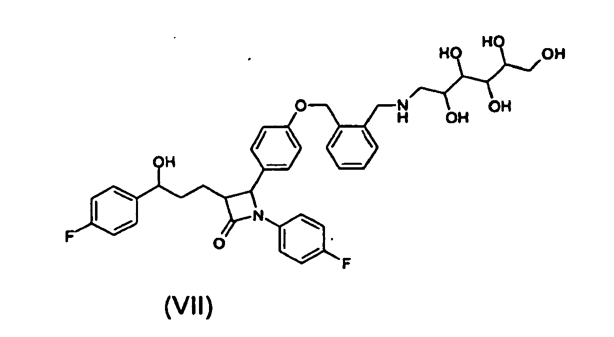

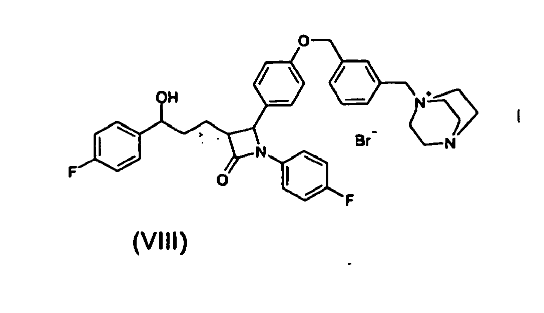

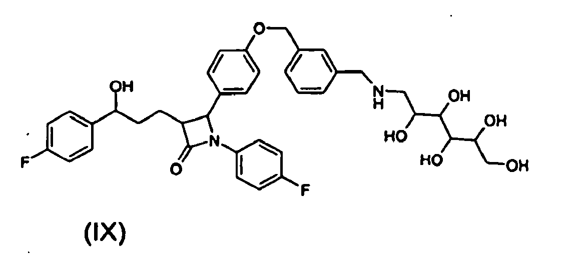

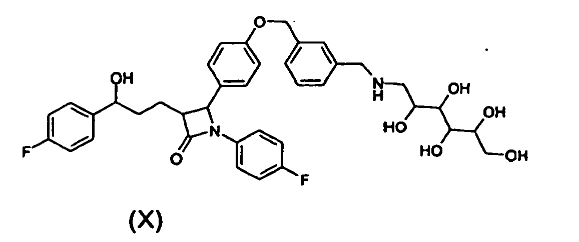

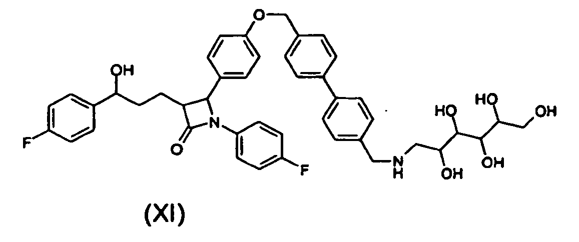

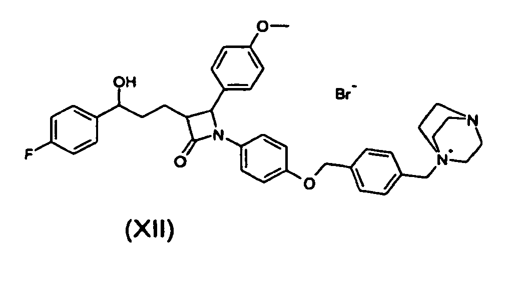

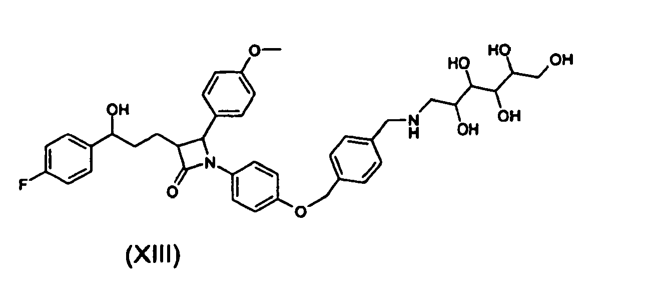

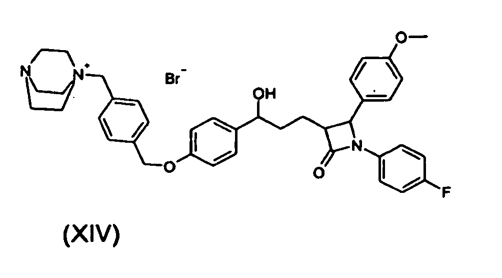

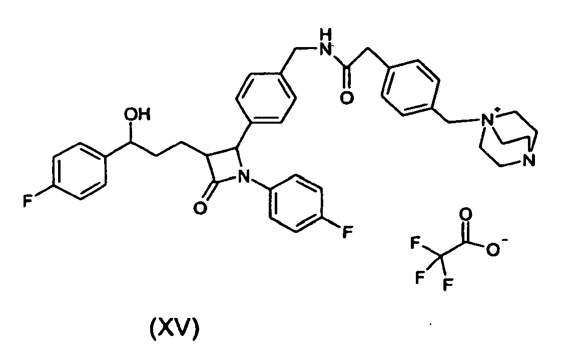

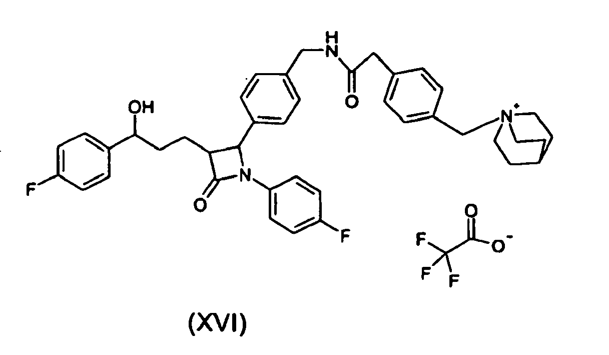

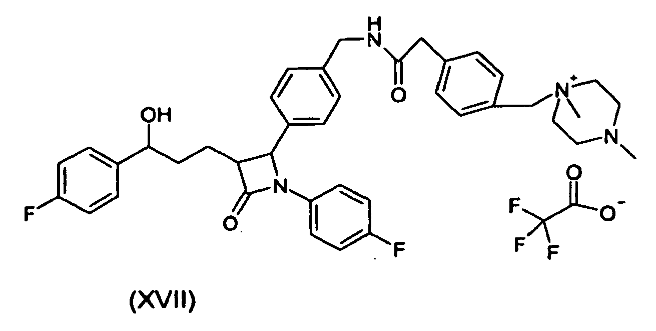

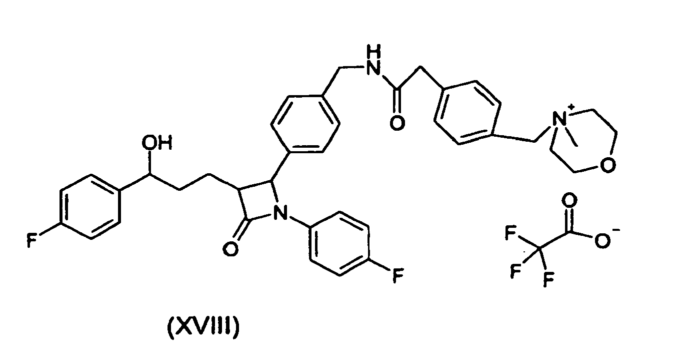

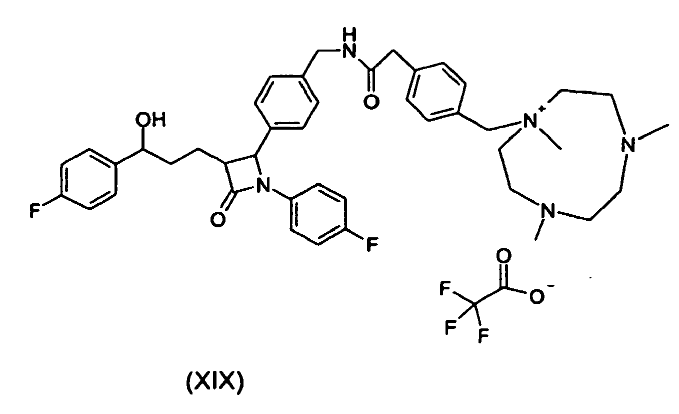

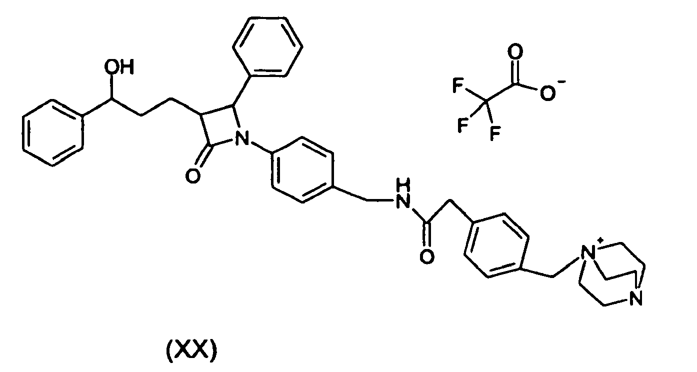

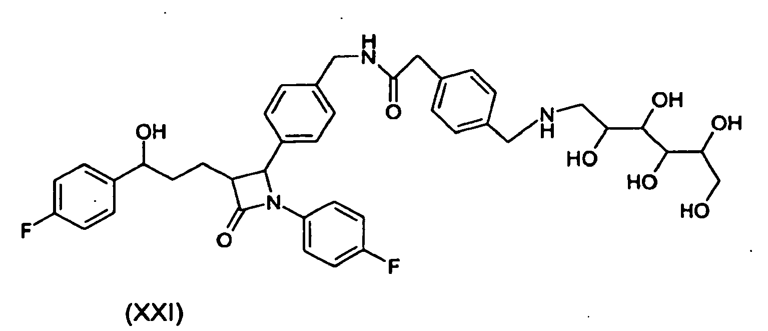

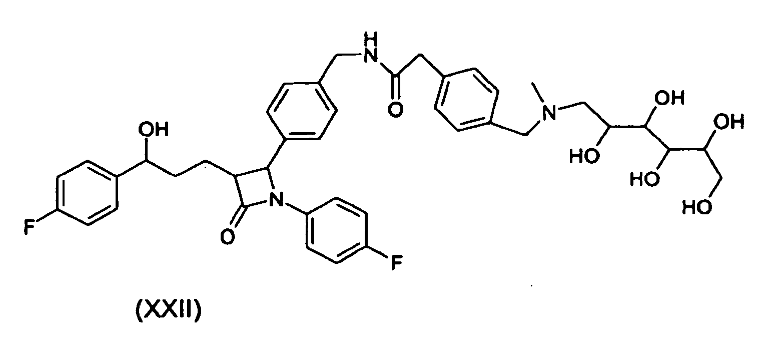

3. Verbindungen der Formel I 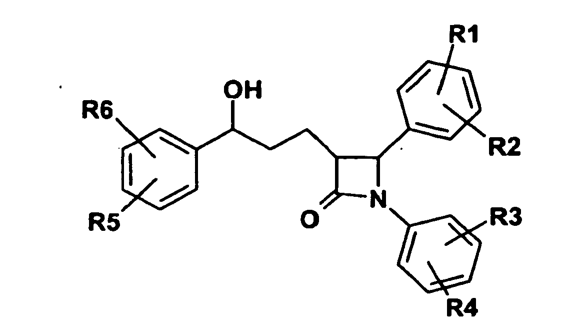 worin bedeuten
| **R1, R2** | **R3, R4** | **R5, R6** | **Salz** |
|---|---|---|---|
| 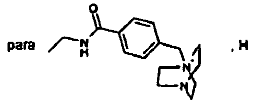 | para-F, H | para-F, H | Cl⁻ |
| 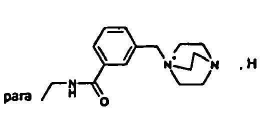 | para-F, H | para-F, H | CF₃COO⁻ |
| 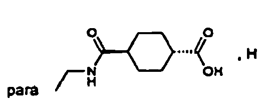 | para-F, H | para-F, H | - |
| 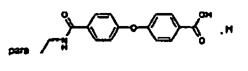 | para-F, H | para-F, H | - |
| para-O-CH₃, H | 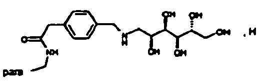 | para-F, H | CF₃COOH |
| para-O-CH₃, H | 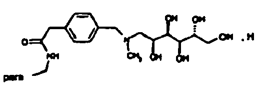 | para-F, H | CF₃COOH |
| 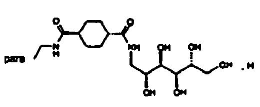 | para-F, H | para-F, H | - |
| 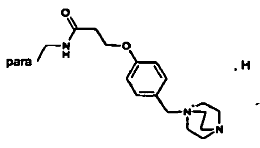 | para-F, H | para-F, H | CF₃COO⁻ |
| 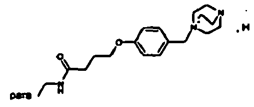 | para-F, H | para-F, H | CF₃COO⁻ |
| 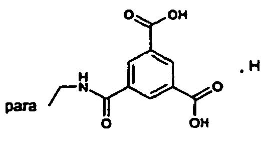 | para-F, H | para-F, H | - |
| 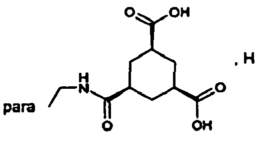 | para-F, H | para-F, H | - |
| 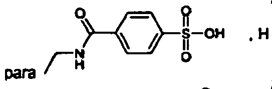 | para-F, H | para-F, H | - |
| 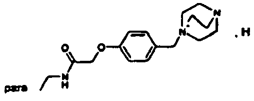 | para-F, H | para-F, H | CF₃COO⁻ |
| para-O-CH₃, H | 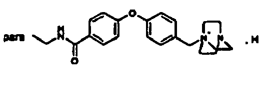 | para-F, H | Cl⁻ |
| para-O-CH₃, H | 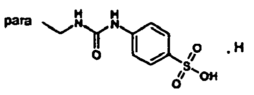 | H, H | - |
| 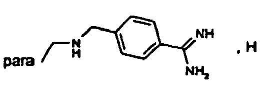 | para-F, H | para-F, H | CF₃COOH |
| 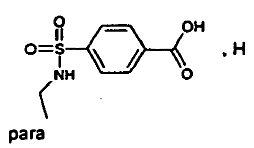 | para-F, H | para-F, H | - |
| para-O-CH₃, H | 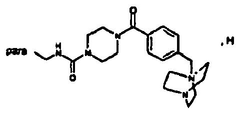 | para-F, H | Cl⁻ |
| para-O-CH₃, H | 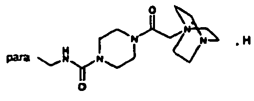 | para-F, H | Cl⁻ |
| para-O-CH₃, H | 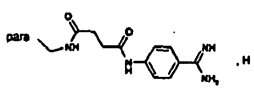 | para-F, H | HCl |
| para-O-CH₃, H | 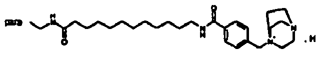 | para-F, H | Br⁻ |
| para-O-CH₃, H | 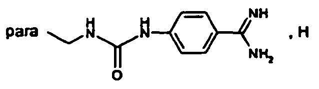 | para-F, H | - |
| para-O-CH₃, H | 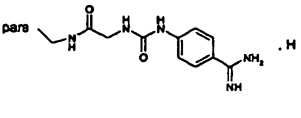 | para-F, H | CF₃COOH |
| para-O-CH₃, H | 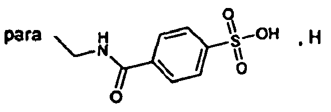 | para-F, H | - |
| para-O-CH₃, H | 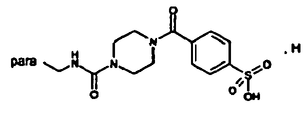 | para-F, H | - |
| para-O-CH₃, H | 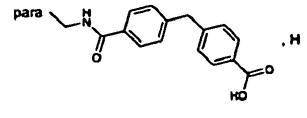 | para-F, H | - |
| para-O-CH₃, H | 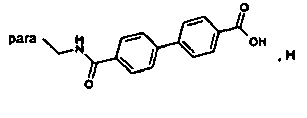 | para-F, H | - |
| para-O-CH₃, H | 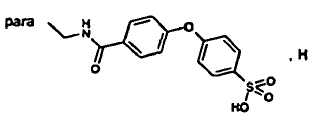 | para-F, H | - |
| para-O-CH₃, H | 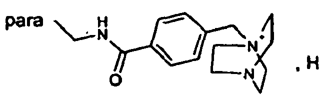 | para-F, H | Cl⁻ |
| para-O-CH₃, H | 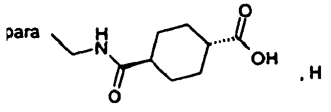 | para-F, H | - |
| para-O-CH₃, H | 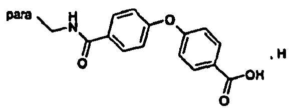 | para-F, H | - |
| para-O-CH₃, H | 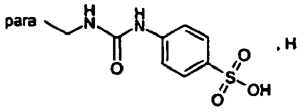 | para-F, H | - |
| para-O-CH₃, H | 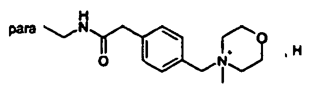 | para-F, H | Br |
| 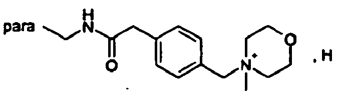 | para-F, H | para-F, H | Br |
| 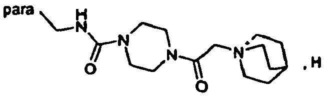 | para-F, H | para-F, H | I⁻ |
| 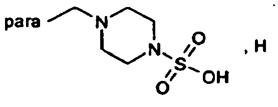 | para-F, H | para-F, H | - |
| 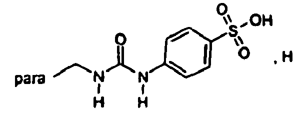 | para-F, H | para-F; H | - |
| para-O-CH₃, H | 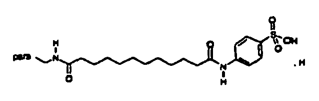 | para-F, H | - |
| para-O-CH₃, H | 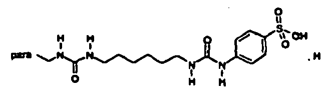 | para-F, H | - |
| para-O-CH3, H | 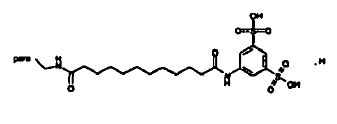 | para-F, H | - |

4. Arzneimittel enthaltend eine oder mehrere der Verbindungen gemäß einem oder mehreren der Ansprüche 1 bis 3.

5. Arzneimittel enthaltend eine oder mehrere der Verbindungen gemäß einem oder mehreren der Ansprüche 1 bis 3 und mindestens einen weiteren Wirkstoff.

6. Arzneimittel, gemäß Anspruch 5, **dadurch gekennzeichnet, daß** es als weiteren Wirkstoff eine oder mehrere Verbindungen, die den Lipidstoffwechsel normalisieren, enthält.

7. Arzneimittel, gemäß Anspruch 5 oder 6, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylhamstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

8. Verbindungen gemäß einem oder mehreren der Ansprüche 1 bis 3 zur Anwendung als Medikament zur Behandlung von Lipidstoffwechselstörungen.

9. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

10. Verwendung der Verbindungen gemäß einem oder mehreren der Ansprüche 1 bis 3 zur Herstellung eines Medikaments zur Behandlung von Hyperlipidämie.

11. Verwendung der Verbindungen gemäß einem oder mehreren der Ansprüche 1 bis 3 zur Herstellung eines Medikaments zur Senkung des Serumcholesterinspiegels.

12. Verwendung der Verbindungen gemäß einem oder mehreren der Ansprüche 1 bis 3 zur Herstellung eines Medikaments zur Behandlung arteriosklerotischer Erscheinungen.

13. Verwendung der Verbindungen gemäß einem oder mehreren der Ansprüche 1 bis 3 zur Herstellung eines Medikaments zur Behandlung von Insulin Resistenz.

## Claims

1. A compound of the formula I, 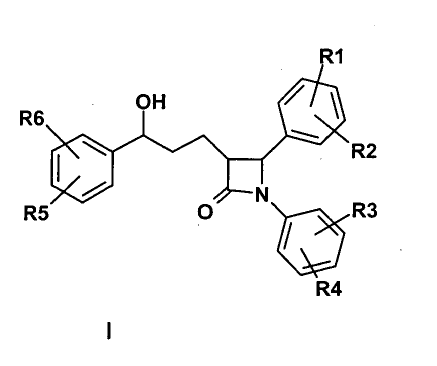 in which
R2, R4, R5, R6 independently of one another are H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, 0-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1, R3 independently of one another are -(CH₂)₀₋₁-NH-(C=O)₀₋₁-(C₀-C₂₅)-alkylene-(C=O)₀₋₁-N(R13)₀₋₁-(LAG) or -(CH₂)₀₋₁(C=O)₀₋₁-NH-(C₀-C₂₅)-alkylene-(C=O)₀₋₁-N(R13)₀₋₁-(LAG); where one or more carbon atoms of the alkylene radical are replaced by aryl or heteroaryl radicals substituted up to three times by R7, or by (C₃-C₁₀)-cycloalkyl or heterocycloalkyl radicals substituted up to three times by R7 and where one or more carbon atoms of the alkylene radical may be replaced by oxygen atoms;
H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 where and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, 0-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R7 is F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
C(NH)(NH₂), NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
LAG is a monosugar residue, an acyclic mono-, di- or tricyclic trialkylammoniumalkyl radical, a sulfonic acid or a carboxylic acid,
R13 is H or CH₃;
and where in each case at least one of the radicals R1 or R3 must have the meaning -(CH₂)₀₋₁-NH-(C=O) ₀₋₁-(C₀-C₂₅)-alkylene-(C=O)₀₋₁-N(R13)₀₋₁-(LAG) or -(CH₂)₀₋₁-(C=O)₀₋₁-NH-(C₀-C₂₅)-alkylene-(C=O)₀₋₁-N(R13)₀₋₁-(LAG) and one or more carbon atoms of the alkylene radical are replaced by up to trisubstituted aryl or heteroaryl radicals or by up to trisubstituted (C₃-C₁₀)-cycloalkyl or heterocycloalkyl radicals and may additionally be replaced by -S(O)ₙ-, where n = 0 - 2, -O-, -(C=O)-, -N(CH₃)-, -N(phenyl)- or -NH-;
and its physiologically acceptable salts.

2. A compound of the formulae I to XXII

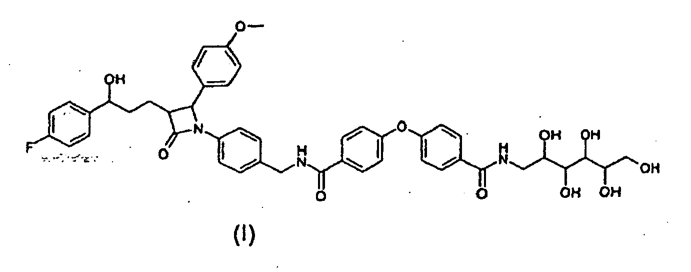

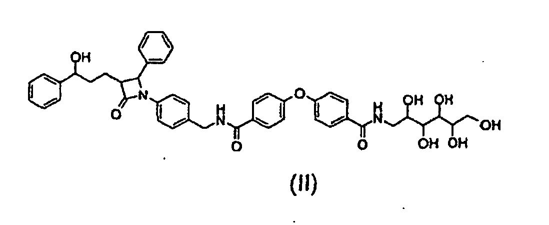

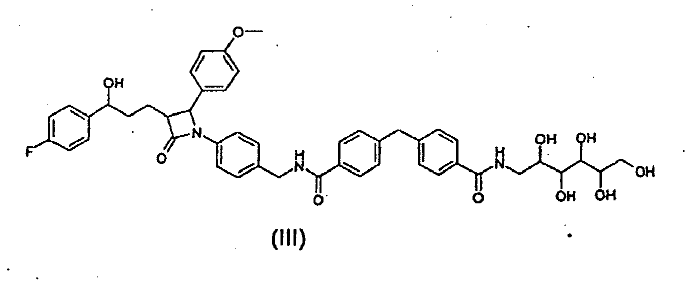

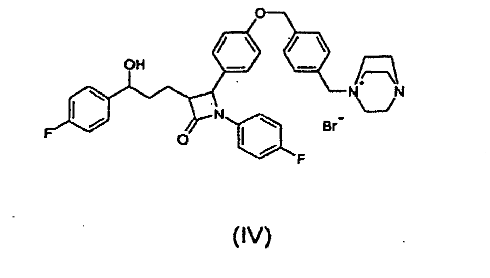

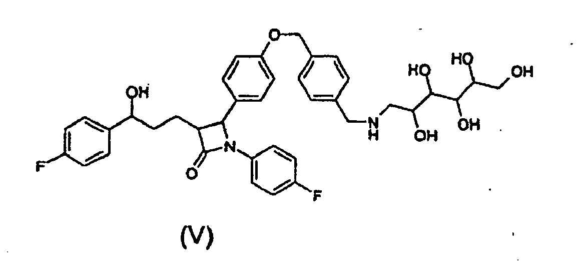

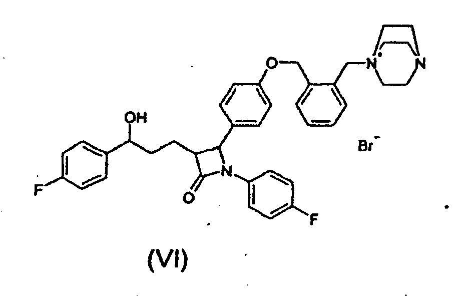

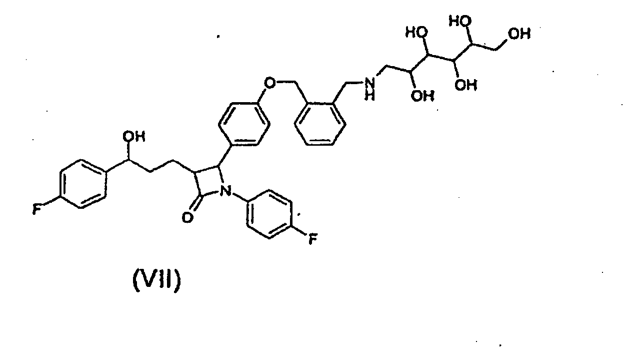

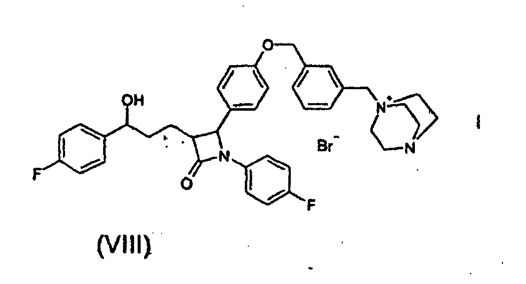

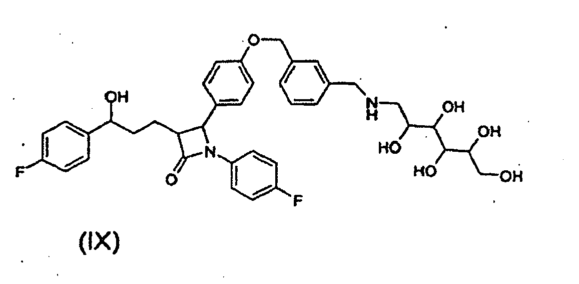

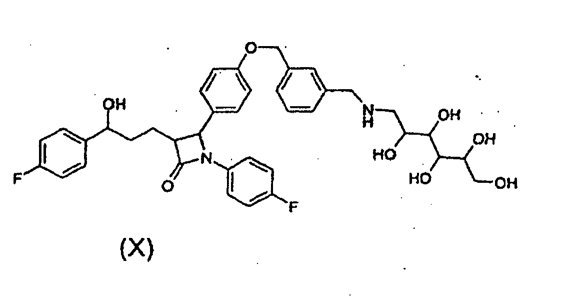

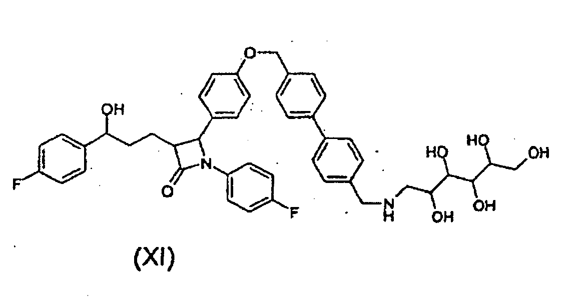

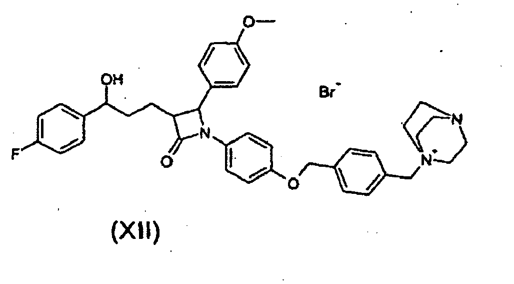

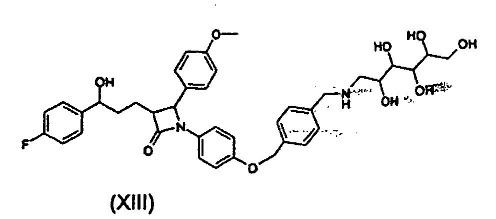

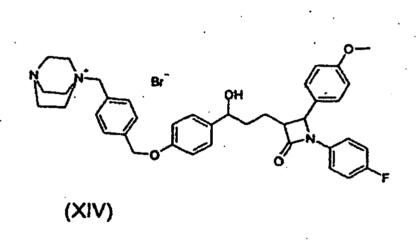

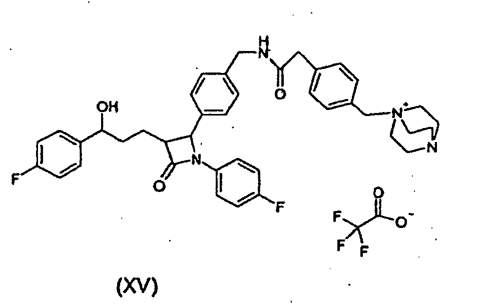

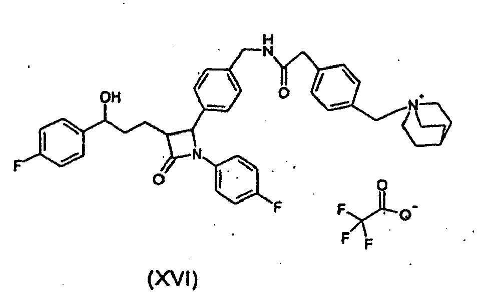

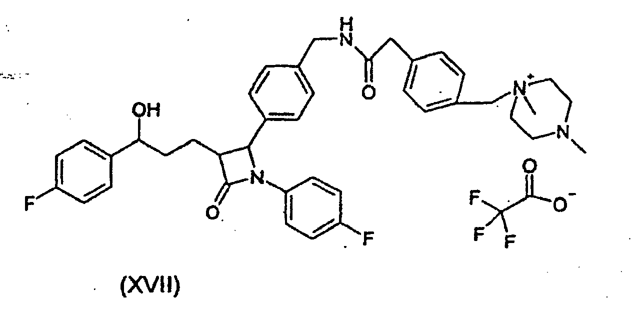

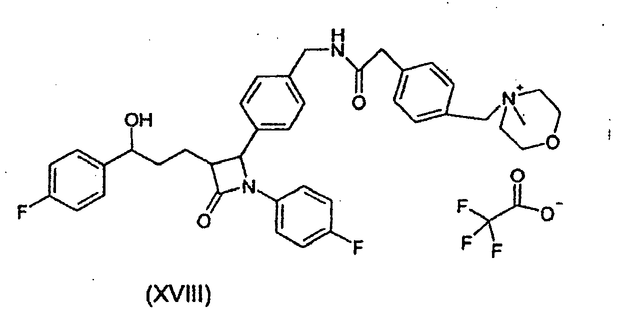

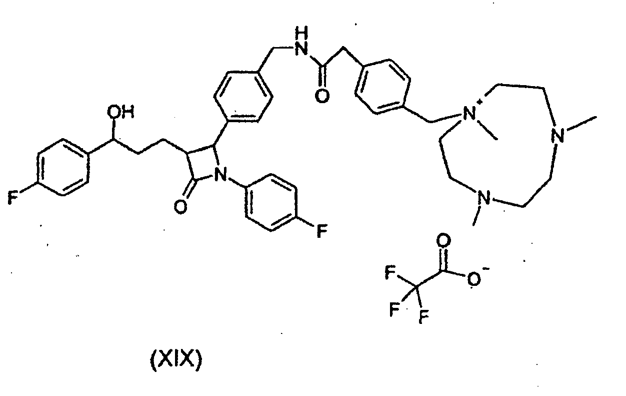

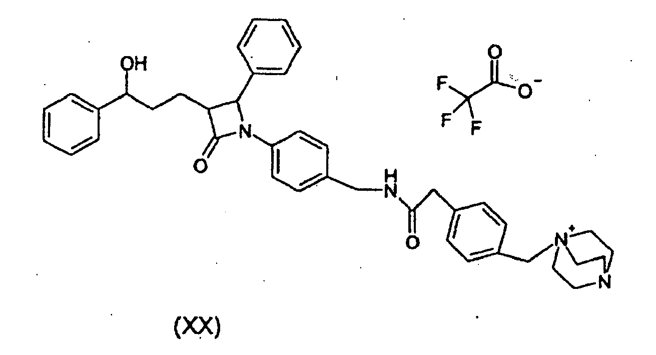

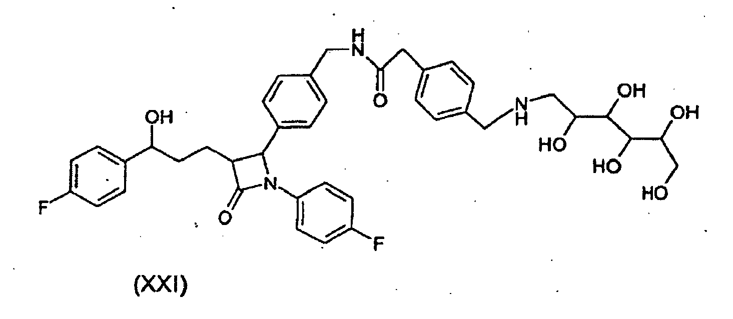

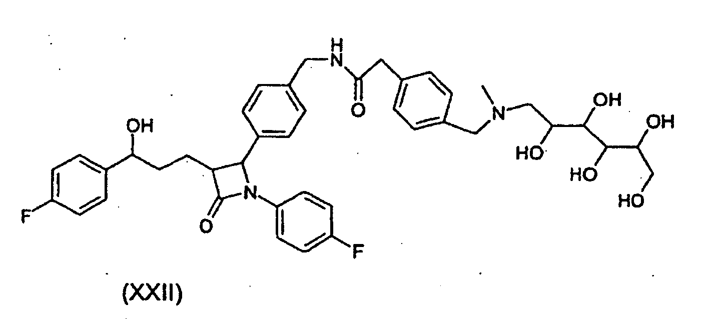

3. A compound of the formula I 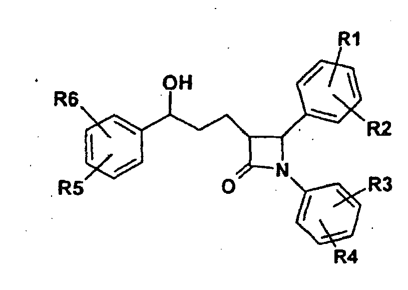 in which the meanings are as follows
| **R1, R2** | **R3, R4** | **R5, R6** | **Salt** |
|---|---|---|---|
| 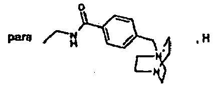 | para-F, H | para-F, H | Cr |
| 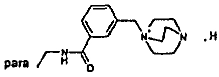 | para-F, H | para-F, H | CF₃COO⁻ |
| 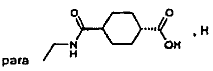 | para-F, H | para-F, H | - |
| 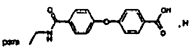 | para-F, H | para-F, H | - |
| para-O-CH₃, H | 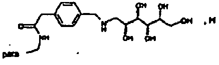 | para-F, H | CF₃COOH |
| para-O-CH₃, H | 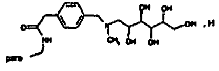 | para-F, H | CF₃COOH |
| 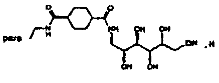 | para-F, H | para-F, H | - |
| 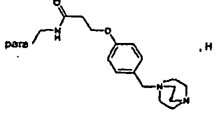 | part-F, H | para-F, H | CF₃COO⁻ |
| 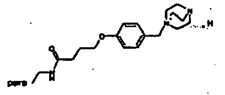 | para-F, H | para-F, H | CF₃COO⁻ |
| 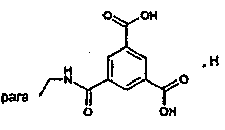 | para-F, H | para-F, H | |
| 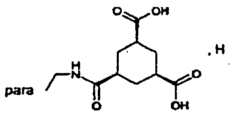 | para-F, H | para-F, H | - |
| 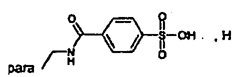 | para-F, H | para-F, H | - |
| 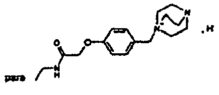 | para-F, H | para-F, H | CF₃COO⁻ |
| para-O-CH₃, H | 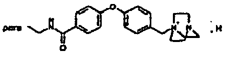 | para-F, H | Cr |
| para-O-CH₃, H | 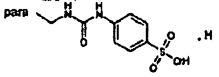 | H, H | - |
| 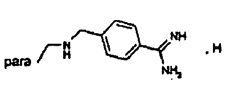 | para-F, H | para-F, H | CF₃COOH |
| 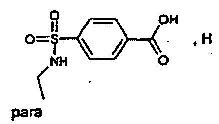 | para-F, H | para-F, H | - |
| para-O-CH₃, H | 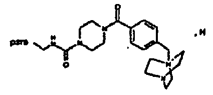 | para-F, H | Cl⁻ |
| para-O-CH₃, H | 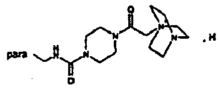 | para-F, H | Cl⁻ |
| para-O-CH₃, H | 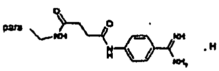 | para-F, H | HCl |
| para-O-CH3, H | 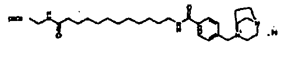 | para-F, H | Br⁻ |
| para-O-CH₃, H | 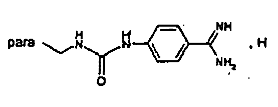 | para-F, H | - |
| para-O-CH₃, H | 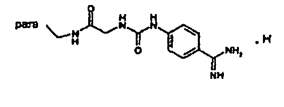 | para-F, H | CF₃COOH |
| para-O-CH₃, H | 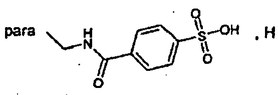 | para-F, H | - |
| para-O-CH₃, H | 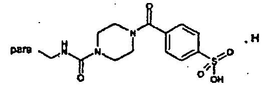 | para-F, H | - |
| para-O-CH₃, H | 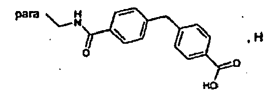 | para-F, H | - |
| para-O-CH₃, H | 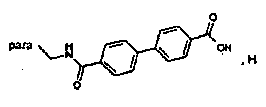 | para-F, H | - |
| para-O-CH₃, H | 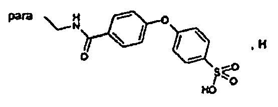 | para-F, H | - |
| para-O-CH₃, H | 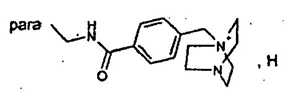 | para-F, H | Cl⁻ |
| para-O-CH₃, H | 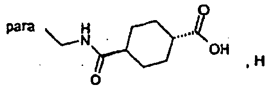 | para-F, H | - |
| para-O-CH₃, H | 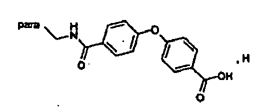 | para-F, H | - |
| para-O-CH₃, H | 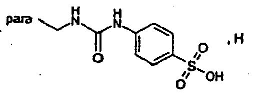 | para-F, H | - |
| para-O-CH₃, H | 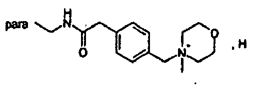 | para-F, H | Br |
| 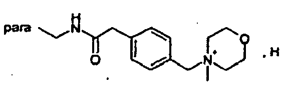 | para-F, H | para-F, H | Br |
| 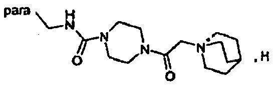 | para-F, H | para-F, H | I⁻ |
| 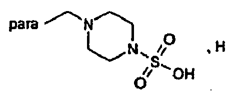 | para-F, H | para-F, H | - |
| 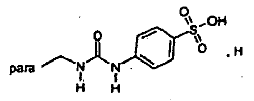 | para-F, H | para-F; | H - |
| para-O-CH₃, H | 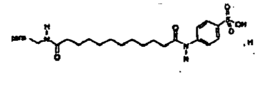 | para-F, H | - |
| para-O-CH₃, H | 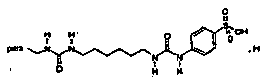 | para-F, H | - |
| para-O-CH3, H | 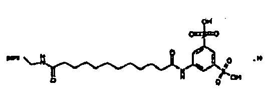 | para-F, H | - |

4. A medicament comprising one or more compounds as claimed in one or more of claims 1 to 3.

5. A medicament comprising one or more compounds as claimed in one or more of claims 1 to 3 and at least one further active compound.

6. The medicament as claimed in claim 5, comprising, as further active compound, one or more compounds which normalize lipid metabolism.

7. The medicament as claimed in claim 5 or 6, which comprises, as further active compound, one or more
antidiabetics, hypoglycemically active compounds, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active compounds which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin-reuptake inhibitors, mixed serotonin and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists (bromocriptine, doprexin), lipase/amylase inhibitors, PPAR modulators, RXR modulators or TR-β-agonists or amphetamines.

8. A compound as claimed in one or more of claims 1 to 3 for use as a medicament for the treatment of impaired lipid metabolism.

9. A process for preparing a medicament comprising one or more of the compounds as claimed in one or more of claims 1 to 3, which comprises mixing the active compound with a pharmaceutically acceptable carrier and bringing this mixture into a form suitable for administration.

10. The use of the compounds as claimed in one or more of claims 1 to 3 for preparing a medicament for treating hyperlipidemia.

11. The use of the compounds as claimed in one or more of claims 1 to 3 for preparing a medicament for lowering the serum cholesterol concentration.

12. The use of the compounds as claimed in one or more of claims 1 to 3 for preparing a medicament for treating arteriosclerotic manifestations.

13. The use of the compounds as claimed in one or more of claims 1 to 3 for preparing a medicament for treating insulin resistance.

## Revendications

1. Composés de formule I, 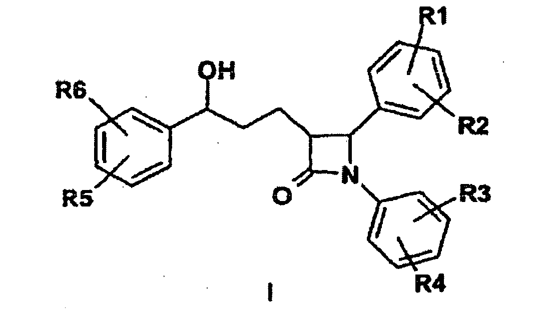 dans laquelle
R2, R4, R5, R6 signifient indépendamment l'un de l'autre H, F, Cl, Br, I, CF₃, NO₂, N₃, CN, COOH, COO(C₁-C₆)alkyle, CONH₂, CONH(C₁-C₆)alkyle, CON[(C₁-C₆)alkyle]₂, (C₁-C₆)-alkyle, (C₂-C₆)-alcényle, (C₂-C₆)-alcynyle, O-(C₁-C₆)-alkyle, où, dans les radicaux alkyle, un, plusieurs ou tous les atomes d'hydrogène peuvent être remplacés par fluor ;
SO₂-NH₂, SO₂NH(C₁-C₆)-alkyle, SO₂N[(C₁-C₆)-alkyle]₂, S-(C₂-C₆)-alkyle, S-(CH₂)ₙ-phényle, SO-(C₁-C₆)-alkyle, SO-(CH₂)ₙ-phényle, SO₂-(C₁-C₆)-alkyle, SO₂-(CH₂)ₙ-phényle, où n = 0-6 et le radical phényle peut être jusqu'à disubstitué par F, Cl, Br, OH, CF₃, NO₂, CN, OCF₃, O-(C₁-C₆)-alkyle, (C₁-C₆)-alkyle, NH₂ ;
NH₂, NH-(C₁-C₆)-alkyle, N((C₁-C₆)-alkyle)₂, NH(C₁-C₇)-acyle, phényle, O-(CH₂)ₙ-phényle, où n = 0-6, où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
R1, R3 signifient, indépendamment l'un de l'autre, -(CH₂)₀₋₁-NH-(C=O)₀₋₁-(C₀-C₂₅)-alkylène-(C=O)₀₋₁- N(R13)₀₋₁-(LAG) ou -(CH₂)₀₋₁-(C=O)₀₋₁-NH- (C₀-C₂₅)-alkylène-(C=O)₀₋₁-N(R13)₀₋₁-(LAG) ; où un ou plusieurs atomes de carbone du radical alkylène sont remplacés par des radicaux aryle ou hétéroaryle jusqu'à trisubstitués par R7 ou par des radicaux (C₃-C₁₀)-cycloalkyle ou hétérocycloalkyle jusqu'à trisubstitués par R7 et où un ou plusieurs atomes de carbone du radical alkylène peuvent être remplacés par des atomes d'oxygène ;
H, F, Cl, Br, I, CF₃, NO₂, N₃, CN, COOH, COO(C₁-C₆)alkyle, CONH₂, CONH(C₁-C₆)alkyle, CON[(C₁-C₆)alkyle]₂, (C₁-C₆)-alkyle, (C₂-C₆)-alcényle, (C₂-C₆)-alcynyle, O-(C₁-C₆)-alkyle, où, dans les radicaux alkyle, un, plusieurs ou tous les atomes d'hydrogène peuvent être remplacés par fluor ; SO₂-NH₂, SO₂NH(C₁-C₆)-alkyle, SO₂N[(C₁-C₆)-alkyle]₂, S-(C₁-C₆)-alkyle, S- (CH₂)ₙ-phényle, SO-(C₁-C₆)-alkyle, SO- (CH₂)n-phényle, SO₂-(C₁-C₆)-alkyle, SO₂-(CH₂)ₙ-phényle, où n = 0-6 et le radical phényle peut être jusqu'à disubstitué par F, Cl, Br, OH, CF₃, NO₂, CN, OCF₃, O-(C₁-C₆)-alkyle, (C₁-C₆)-alkyle, NH₂ ;
NH₂, NH-(C₁-C₆)-alkyle-, N((C₁-C₆)-alkyle)₂, NH(C₁-C₇-acyle, phényle, O-(CH₂)ₙ-phényle, où n = 0-6, où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ; CON[(C₁-C₆)alkyle]₂, (C₁-C₆)-alkyle, (C₂-C₆)-alcényle, (C₂-C₆)-alcynyle, O-(C₁-C₆)-alkyle, où, dans les radicaux alkyle, un, plusieurs ou tous les atomes d'hydrogène peuvent être remplacés par fluor ;
PO₃H₂, SO₃H, SO₂-NH₂, SO₂NH(C₁-C₆)-alkyle, SO₂N[(C₁-C₆)-alkyle]₂, S-(C₁-C₆)-alkyle, S-(CH₂)ₙ-phényle, SO-(C₁-C₆)-alkyle, SO-(CH₂)ₙ-phényle, SO₂-(C₁-C₆)-alkyle, SO₂-(CH₂)ₙ-phényle, où n = 0-6 et le radical phényle peut être jusqu'à disubstitué par F, Cl, Br, OH, CF₃, NO₂, CN, OCF₃, O-(C₁-C₆)-alkyle, (C₁-C₆)-alkyle, NH₂ ;
C(NH)(NH₂), NH₂, NH-(C₁-C₆)-alkyle, N((C₁-C₆)-alkyle)_{2,} NH(C₁-C₇)-acyle, phényle, O-(CH₂)ₙ-phényle, où n = 0-6, où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
LAG signifie un résidu monosucré, un radical trialkylammoniumalkyle acyclique, monocyclique, bicyclique ou tricyclique, un acide sulfonique ou un acide carboxylique,
R13 signifie H ou CH₃ ;
et où au moins un des radicaux R1 ou R3 signifie toujours
-(CH₂)₀₋₁-NH-(C=O)₀₋₁-(C₀-C₂₅)-alkylène-(C=O)₀₋₁-N(R13)₀₋₁-(LAG) ou
-(CH₂)₀₋₁-(C=O)₀₋₁-NH-(C₀-C₂₅)-alkylène-(C=O)₀₋₁-N(R13)₀₋₁-(LAG) et un ou plusieurs atomes de carbone du radical alkylène sont remplacés par des radicaux aryle ou hétéroaryle jusqu'à trisubstitués ou par des radicaux (C₃-C₁₀)-cycloalkyle ou hétérocycloalkyle jusqu'à trisubstitués et peuvent en outre être remplacés par -S(O)ₙ-, avec n = 0-2, -O-, -(C=O)-, -N(CH₃)-, -N(phényl)- ou -NH-,
ainsi que leurs sels physiologiquement acceptables.

2. Composés des formules I à XXII

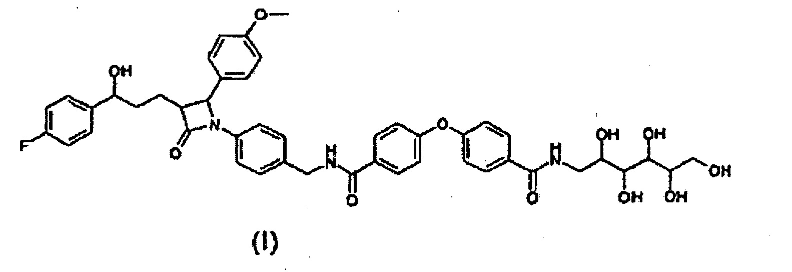

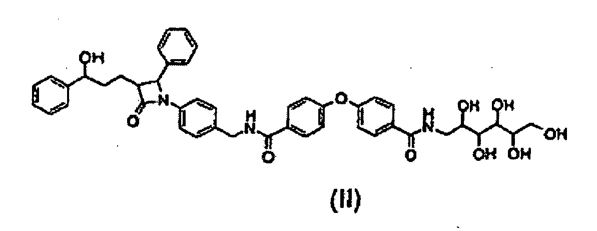

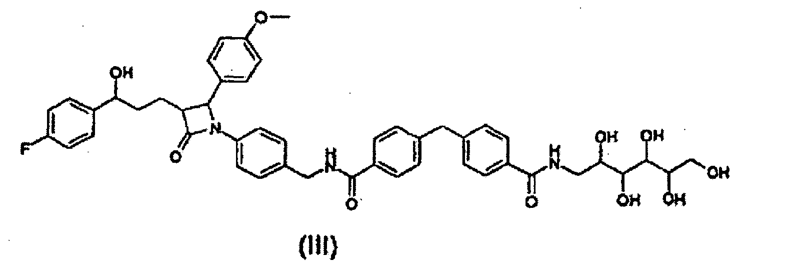

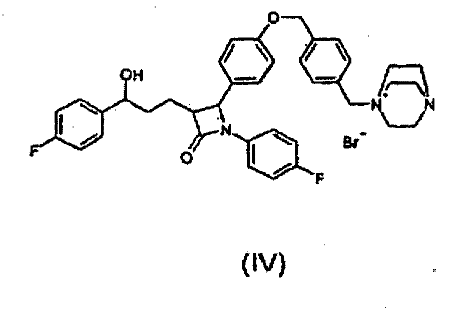

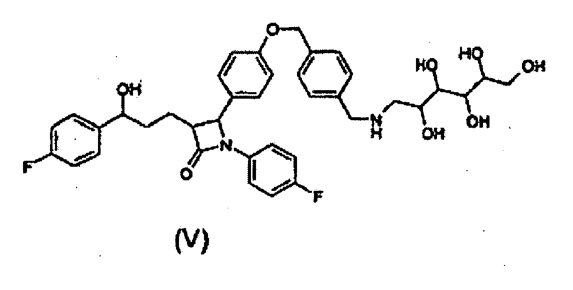

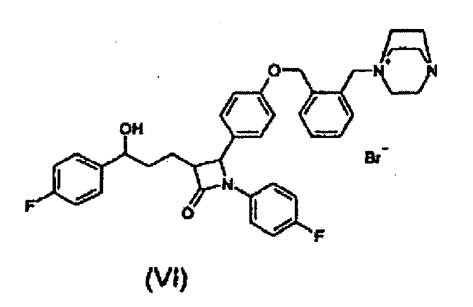

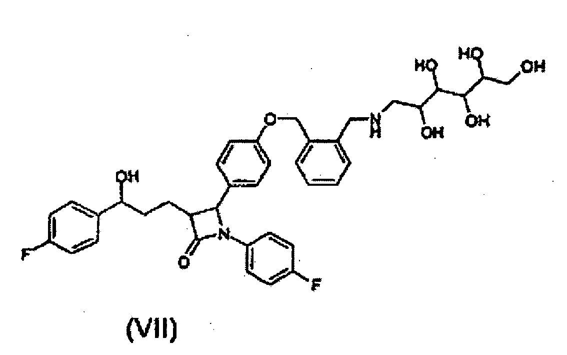

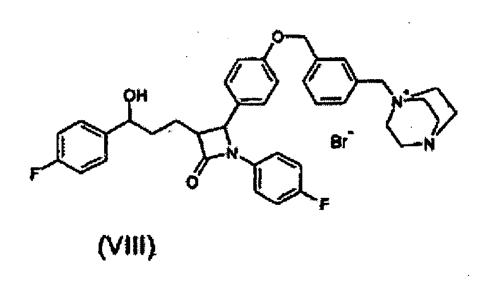

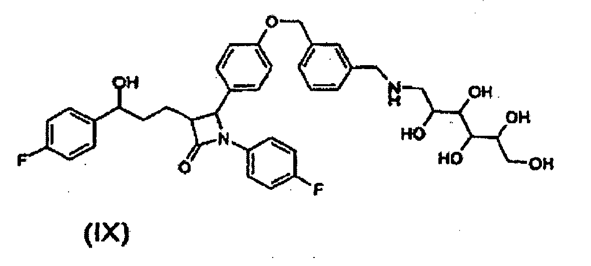

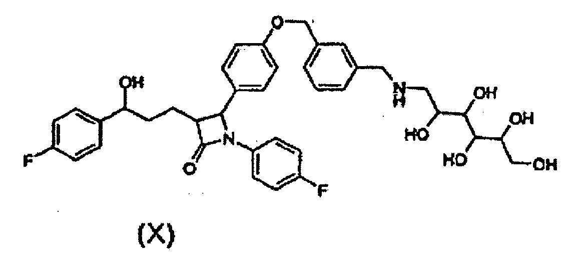

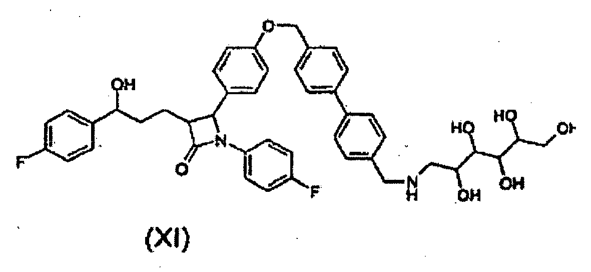

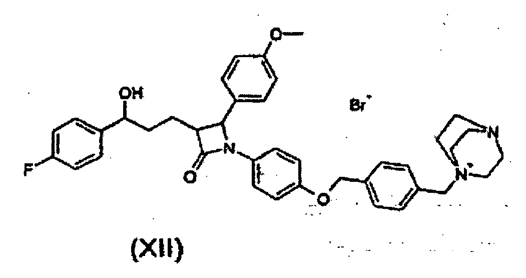

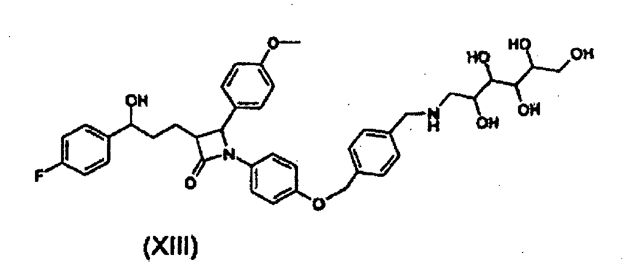

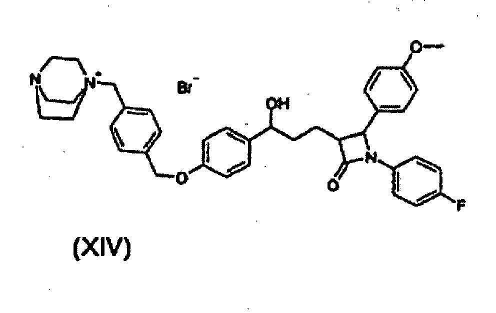

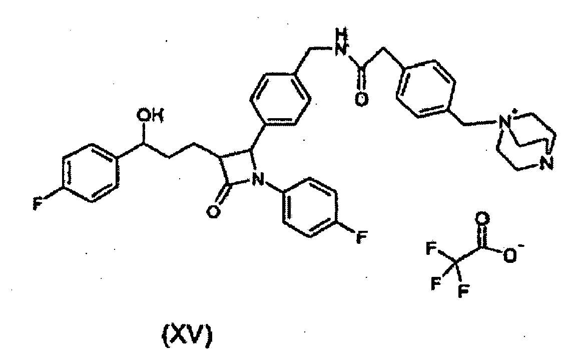

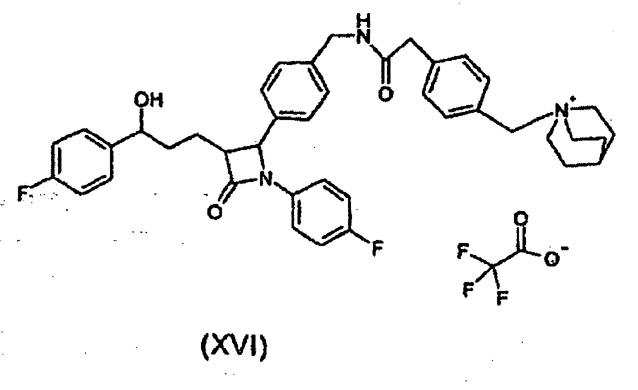

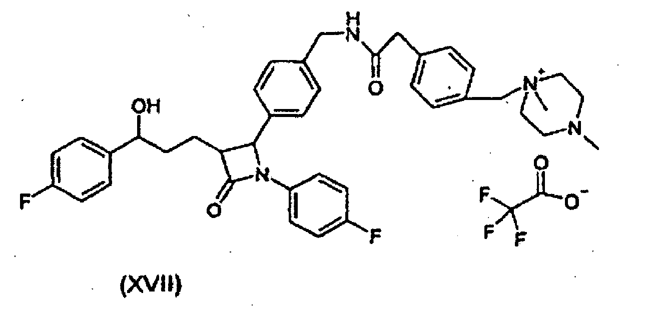

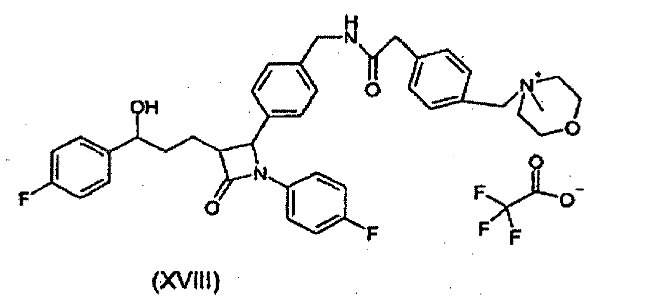

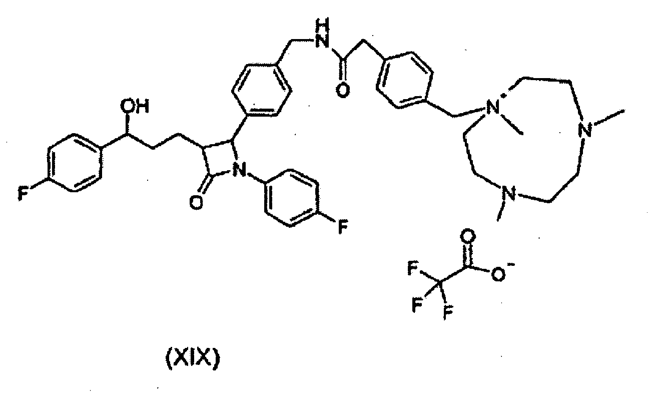

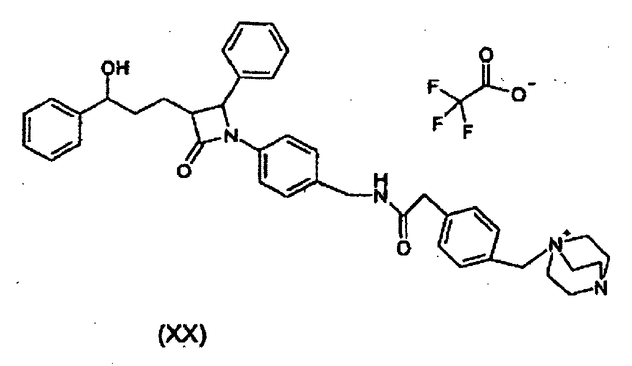

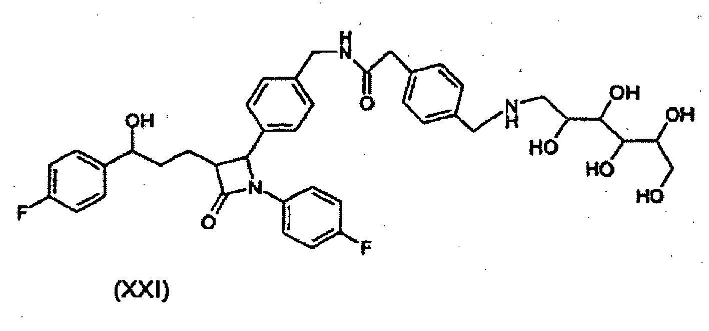

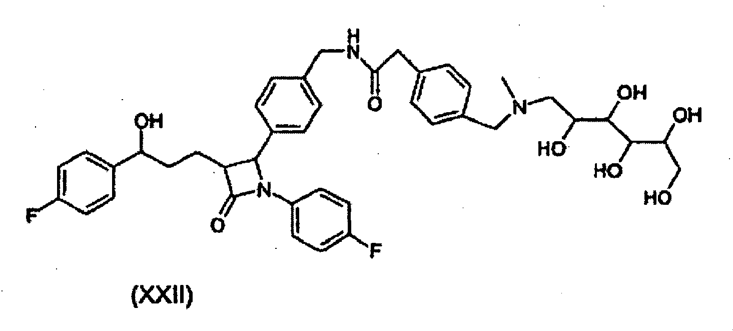

3. Composés de formule I 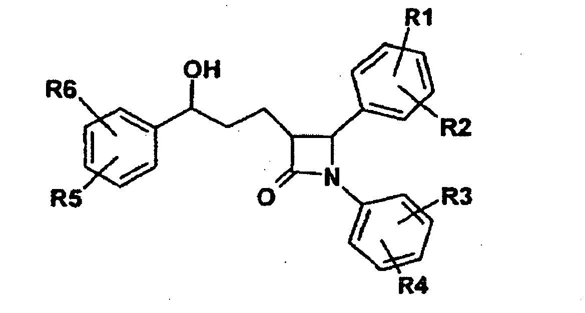 dans laquelle
| **R1, R2** | **R3, R4** | **R5, R6** | **Sel** |
|---|---|---|---|
| 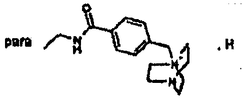 | para-F, H | para-F, H | Cl⁻ |
| 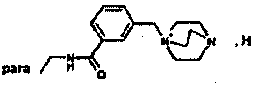 | para-F, H | para-F, H | CF₃COO⁻ |
| 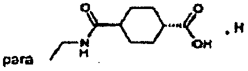 | para-F, H | para-F, H | - |
| 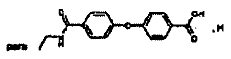 | para-F, H | para-F, H | - |
| para-O-CH₃, H | 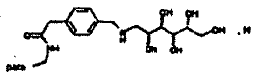 | para-F, H | CF₃COOH |
| para-O-CH₃, H | 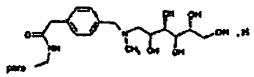 | para-F, H | CF₃COOH |
| 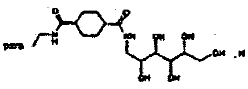 | para-F, H | para-F, H | - |
| 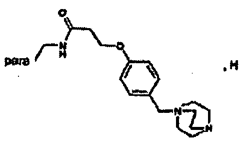 | para-F, H | para-F, H | CF₃COO⁻ |
| 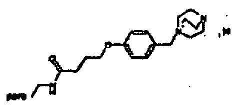 | para-F, H | para-F, H | CF₃COO⁻ |
| 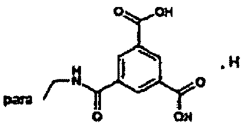 | para-F, H | para-F, H | - |
| 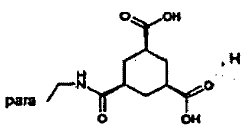 | para-F, H | para-F, H | - |
| 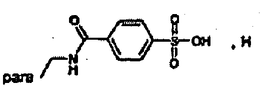 | para-F, H | para-F, H | - |
| 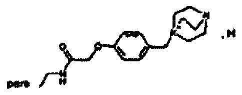 | para-F, H | para-F, H | CF₃COO⁻ |
| para-O-CH₃, H | 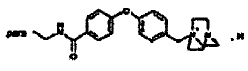 | para-F, H | Cl⁻ |
| para-O-CH₃, H | 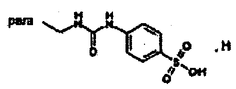 | H, H | - |
| 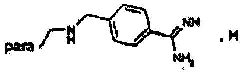 | para-F, H | para-F, H | CF₃COOH |
| 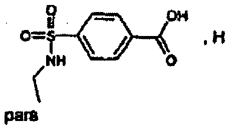 | para-F, H | para-F, H | - |
| para-O-CH₃, H | 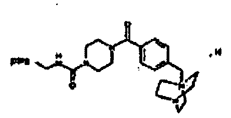 | para-F, H | Cl⁻ |
| para-O-CH₃, H | 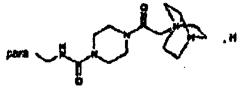 | para-F, H | Cl⁻ |
| para-O-CH₃, H | 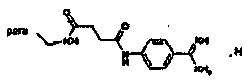 | para-F, H | HCl |
| para-O-CH₃, H | 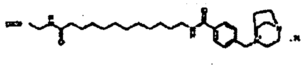 | para-F, H | Br⁻ |
| para-O-CH₃, H | 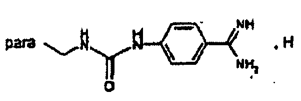 | para-F, H | - |
| para-O-CH₃, H | 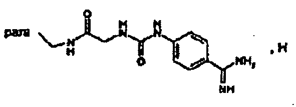 | para-F, H | CF₃COOH |
| para-O-CH₃, H | 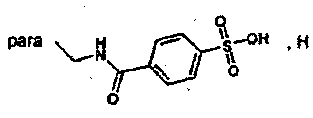 | para-F, H | - |
| para-O-CH₃, H | 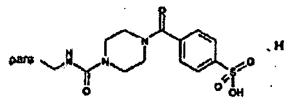 | para-F, H | - |
| para-O-CH₃, H | 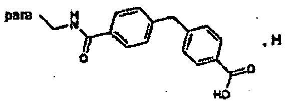 | para-F, H | - |
| para-O-CH₃, H | 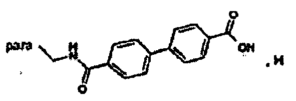 | para-F, H | - |
| para-O-CH₃, H | 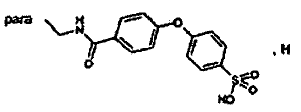 | para-F, H | - |
| para-O-CH₃, H | 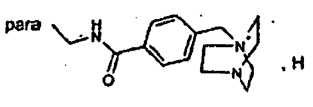 | para-F, H | Cl⁻ |
| para-O-CH₃, H | 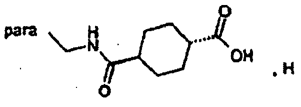 | para-F, H | - |
| para-O-CH₃, H | 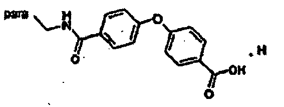 | para-F, H | - |
| para-O-CH₃, H | 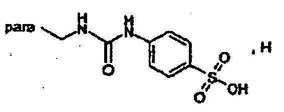 | para-F, H | - |
| para-O-CH₃, H | 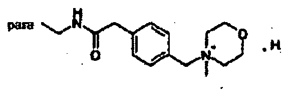 | para-F, H | Br |
| 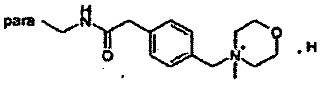 | para-F, H | para-F, H | Br |
| 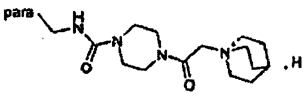 | para-F, H | para-F, H | I⁻ |
| 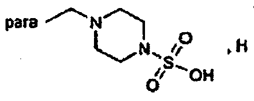 | para-F, H | para-F, H | - |
| 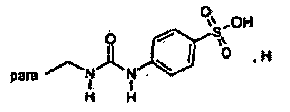 | para-F, H | para-F; | H - |
| para-O-CH₃, H | 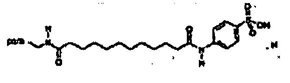 | para-F, H | - |
| para-O-CH₃, H | 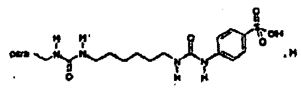 | para-F, H | - |
| para-O-CH3, H | 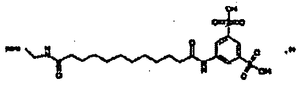 | para-F, H | - |

4. Médicament contenant un ou plusieurs des composés selon l'une ou plusieurs des revendications 1 à 3.

5. Médicament contenant un ou plusieurs des composés selon l'une ou plusieurs des revendications 1 à 3 et au moins une autre substance active.

6. Médicament selon la revendication 5, **caractérisé en ce qu'**il contient, comme autre substance active, un ou plusieurs composés qui normalisent le métabolisme des lipides.

7. Médicament selon la revendication 5 ou 6, **caractérisé en ce qu'**il contient comme autre substance active un(e) ou plusieurs antidiabétiques, substances actives hypoglycémiques, inhibiteurs de HMGCoA-réductase, inhibiteurs de la résorption de cholestérol, agonistes de PPAR gamma, agonistes de PPAR alpha, agonistes de PPAR alpha/gamma, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène synthétase, antagonistes de la lipoprotéine (a), inhibiteurs de lipase, insulines, sulfonylurées, biguanides, méglitinides, thiazolidinediones, inhibiteurs d'α-glucosidase, substances actives agissant sur le canal potassique dépendant de l'ATP des cellules bêta, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, agonistes de la MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, antagonistes de galanine, hormones de croissance, composés libérant les hormones de croissance, agonistes de TRH, modulateurs 2 ou 3 de la protéine découplante, agonistes de la leptine, agonistes de DA (bromocriptine, doprexine), inhibiteurs de lipase/amylase, modulateurs de PPAR, modulateurs de RXR ou agonistes de TR-β ou amphétamines.

8. Composés selon l'une ou plusieurs des revendications 1 à 3 pour une utilisation comme médicament destiné au traitement de troubles du métabolisme des lipides.

9. Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.

10. Utilisation des composés selon l'une ou plusieurs des revendications 1 à 3 pour la préparation d'un médicament destiné au traitement de l'hyperlipidémie.

11. Utilisation des composés selon l'une ou plusieurs des revendications 1 à 3 pour la préparation d'un médicament destiné à abaisser le niveau de cholestérol dans le sérum.

12. Utilisation des composés selon l'une ou plusieurs des revendications 1 à 3 pour la préparation d'un médicament destiné au traitement de phénomènes artérioscléreux.

13. Utilisation des composés selon l'une ou plusieurs des revendications 1 à 3 pour la préparation d'un médicament destiné au traitement de la résistance à l'insuline.
